# EUROPEAN PATENT SPECIFICATION

(11) **EP 2 155 880 B1**
(45) Date of publication and mention of the grant of the patent: **10.08.2016**
(21) Application number: 08772802.8
(22) Date of filing: 13.06.2008
(51) Int. Cl.: C12N 15/62, A01H 5/00, A01H 5/10, C07K 19/00, C12N 15/54, C12N 15/82, C12N 5/10, C12N 9/10, C12P 21/02, C12P 21/08

(54) **MODIFYING GLYCOPROTEIN PRODUCTION IN PLANTS**
MODIFIKATION DER GLYKOPROTEINPRODUKTION IN PFLANZEN
MODIFICATION DE LA PRODUCTION DE GLYCOPROTÉINE DANS DES PLANTES

(30) Priority: 15.06.2007 US 944344 P
(43) Date of publication of application: 24.02.2010
(73) Proprietor: Medicago Inc., Sainte-Foy, QC G1V 3V9 (CA); Centre National de la Recherche Scientifique, 75794 Paris Cedex 16 (FR); UNIVERSITE DE ROUEN, 76821 Mont-Saint-Aignan Cédex (FR)
(72) Inventor: D'AOUST, Marc-Andre, Quebec, Quebec G1X 4N4 (CA); MARQUET-BLOUIN, Estelle, F-76270 Saint-Martin L'Hortier (FR); BARDOR, Muriel, F-76230 Isneauville (FR); BUREL, Carole, F-76760 Bourdainville (FR); FAYE, Loic, F-76160 Saint Jacques sur Darnetal (FR); LEROUGE, Patrice, F-76530 GRAND COURONNE (FR); VEZINA, Louis-Philippe, Neuville, Québec G0A 2R0 (CA); GOMORD, Véronique, Parc Vivaldi F-76000 Rouen (FR); AQUIN, Stéphanie, CH-1024 Ecublens (CH); RIHOUEY, Christophe, Sainte Foy, Quebec G1V 3V9 (CA); PACCALET, Thomas, Quebec, Quebec G1R 2C2 (CA); SOURROUILLE, Christophe, F-94100 Saint Maur des Fosses (FR)
(74) Representative: van Kooij, Adriaan
(86) International application number: PCT/CA2008/001139
(87) International publication number: WO 2008/151440

(56) References cited:
- WO-A1-01/31045
- WO-A2-01/29242
- WO-A2-03/078637
- WO-A2-2004/065540
- US-A1- 2005 223 430
- US-B1- 7 125 978
- OKADA K ET AL: "EXPRESSION AND INTEGRATION OF GENES INTRODUCED INTO HIGHLY SYNCHRONIZED PLANT PROTOPLASTS" MGG - MOLECULAR GENETICS AND GENOMICS, SPRINGER, BERLIN, DE, vol. 205, no. 3, 1 January 1986 (1986-01-01), pages 398-403, XP001246711 ISSN: 1617-4615
- SOURROUILLE C. ET AL.: 'Down-regulated expression of plant-specific glycoepitopes in alfalfa' PLANT BIOTECHNOL. J. vol. 6, no. 7, September 2008, pages 702 - 721, XP008125384
- FERRARA C. ET AL.: 'Modulation of therapeutic antibody effector functions by glycosylation engineering: influence of Golgi enzyme localization domain and co-expression of heterologous beta1, 4-N-acetylglucosaminyltransferase III and Golgi alpha-mannosidase II' BIOTECHNOL. BIOENG. vol. 93, no. 5, April 2006, pages 851 - 861, XP009082743
- SAINT-JORE-DUPAS C. ET AL.: 'From planta to pharma with glycosylation in the toolbox' TRENDS BIOTECHNOL. vol. 25, no. 7, July 2007, pages 317 - 323, XP022116342

## Description

### FIELD OF INVENTION

The present invention relates to methods for modifying glycoprotein production in plants. The present invention also provides plants with modified glycoprotein production.

### BACKGROUND OF THE INVENTION

Immunoglobulins (IgGs) are complex heteromultimeric proteins with characteristic affinity for specific antigenic counterparts of various natures. Today, routine isolation of IgG-producing cell lines, and the advent of technologies for IgG directed evolution and molecular engineering have profoundly impacted their evolution as biotherapeutics and in the general life science market. Therapeutic monoclonal IgG (monoclonal antibodies, mAbs) dominate the current market of new anti-inflammatory and anti-cancer drugs and hundreds of new candidates are currently under research and clinical development for improved or novel applications. The annual market demand for mAbs ranges from a few grams (diagnostics), a few kilograms (anti-toxin) to up to one or several hundreds of kilograms (bio-defense, anti-cancer, anti-infectious, anti-inflammatory).

Although CHO cell culture is still their preferred production host at commercial scale, it is generally accepted that for mAbs to reach their full impact on the life science market, alternative production systems have to be developed, as the facilities required for these cultures are not easily modulated in scale, their building and maintenance costs are extremely high and steadily increasing, and their validation under GMP still requires an average of three years following construction. Even at the early development stages, the selection of CHO cell lines with acceptable yields and productivity remains a costly and long process. New production systems that would decrease the upstream costs (higher yields, simpler technologies and infrastructures), have shorter lead time, be more flexible in capacity, while meeting the current reproducibility, quality and safety features of current cell culture systems are likely to have a significant impact on the development of mAbs and vaccines for the life science market, at every development stages.

Plants are suitable hosts for the production of mAbs and several other proteins which have current applications in life sciences (see Ko and Koprowski 2005; Ma et al., 2005; Yusibov et al., 2006 for recent reviews). MAbs have been produced in stable transgenic plant lines at yields up to 200 mg/kg fresh weight (FW), and through transient expression at rates of up to 20 mg/kg FW (Kathuria, 2002). Giritch et al. (2006) report expression levels of 200-300 mg/kg of leaf weight for an IgG, with one cited maximum of 500 mg/kg through the use of a multi-virus based transient expression system.

WO 03/078637 discloses methods for optimizing glycan processing in organisms (and in particular, plants) so that a glycoprotein having complex type bi-antennary glycans and thus 5 containing galactose residues on both arms and which are devoid of (or reduce in) xylose and fucose can be obtained.

WO 2004/065540 discloses nucleic acid molecules, including fusion constructs, having catalytic activity and the use of same in glycosylation engineering of host cells to generate polypeptides with improved therapeutic properties, including antibodies with increased Fc receptor binding and increased effector function.

WO 01/29242 discloses methods for producing a post-translationally modified heterologous polypeptide in a plant host system by altering the natural post-translational modification abilities of that plant host system. In another aspect of this method, altering the natural post-translational modifications is done by transforming the plant host system with one or more nucleic acid sequences encoding a post-translational modification enzyme.

Plant and mammalian N-glycosylation process differ. The later steps of N-glycosylation in mammalian cells add β1,4galactose, α1,6fucose (beta-1,4galactose, alpha-1,6fucose) and terminal sialic acid residues to complex glycans. However, in plants β1,3galactose, α1,3fucose (beta-1,3galactose, alpha-1,3fucose), α1,4fucose and β1,2xylose (alpha-1,4fucose and beta-1,2xylose) residues are added. Alpha-1,3fucose and β1,2xylose being constituents of glyco-epitopes of some plant allergens, these residues are considered potentially immunogenic and their occurrence on therapeutic proteins, including antibodies is undesired.

The addition of a KDEL sequence to the C-Terminal of a peptide is typically used to ensure retrieval of the peptide from the Golgi back to the ER. This approach has been used to produce a non-fucosylated and non-xylosylated antibody using agroinfiltration of tobacco leaves (Sriraman et al., 2004). However, the added KDEL peptide is potentially immunogenic, and this approach has limited applicability to the production of therapeutic proteins.

Control of α1,3fucose (alpha-1,3fucose) and β1,2xylose (beta-1,2xylose) addition has also been attained by modifying expression of fucosyltransferase and xylosyltransferase. Mutants lacking the capacity to add α1,3fucose and β1,2xylose on complex glycans have been produced in a moss (*Plyscomitrella patens*; Koprivova et al., 2004) and *Arabidopsis thaliana* (Strasser et al., 2004). Partial inhibition of plant fucosyltransferase and xylosyltransferase expression has also been achieved by expression of interfering RNAs (RNA*i*)targeted to α1,3fucosyltransferase and β1,2xylosyltransferase genes in *Lemna minor* (Cox et al., 2006). However complete inhibition of these enzymatic activities have deleterious effects on several plant species, since they interfere with key developmental events such as pollen formation or seed set. RNA*i*-induced specific degradation of mRNA may not be stable over time, and it may not applicable to a large spectrum of plant-based platforms used for the production of therapeutics since it has been reported to be sensitive to environmental factors.

WO 03/078637 discloses the use of human galactosyltransferase to catalyze the addition of terminal β1,4 galactose (GalT) on plant glycans. Expression of GalT, and targeting of its activity to the cis Golgi through the use of a fusion with the transmembrane domain of a xylosyltransferase resulted in the addition of terminal galactose and a decrease in N-glycans bearing plant specific residues (see also Bakker et al., 2006). Breeding these plants with plants containing a recombinant IgG resulted in a significant but variable decrease in glycans containing fucose and xylose.

The attachment of a beta-1,4-linked N-acetylglucosamine (GlnNAc) residue to beta-linked mannose to produce a bisected GlcNac is catalyzed by N-acetylglucosaminyltrasnferase III (GnT-III; EC 2.4.1.144). Introduction of this enzyme into plants has been described (Rouwendal et al., 2007), and plants expressing GnT-III comprised proteins with complex N-glycans were bisected and carried two GlnAc residues.

### SUMMARY OF THE INVENTION

The present invention relates to methods of modifying glycoprotein production in plants. The present invention also provides plants with modified glycoprotein production.

It is an object of the invention to provide an improved method for modifying glycoprotein production in plants.

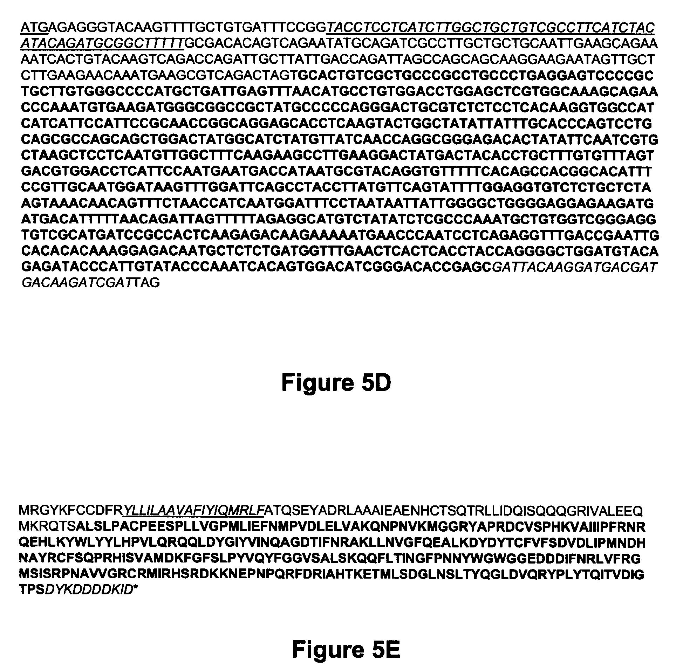

There is provided herein a nucleic acid having a nucleotide sequence (A) comprising nucleotides 1-1062 of SEQ ID NO:17 (GNT1-GalT; Figure 5d), or comprising a nucleotide sequence that exhibits from about 80% to 100% identity with the nucleotides 1-1077 of SEQ ID NO:17, as determined using the following parameters: Program: blastn; Database: nr; Expect 10; filter: low complexity; Alignment: pairwise; Word size: 11, wherein the nucleotide sequence encodes a protein that modifies glycosylation of a protein of interest.

Also disclosed is a nucleic acid having a nucleotide sequence (B) comprising a first nucleic acid sequence comprising nucleotides 5-1198 of SEQ ID NO:14 (GalT), or comprising a sequence that exhibits from about 80% to 100% identity with the nucleotides 5-1198 of SEQ ID NO:14, as determined using the following parameters: Program: blastn; Database: nr; Expect 10; filter: low complexity; Alignment: pairwise; Word size: 11, wherein the first nucleic acid sequence encodes a protein that modifies glycosylation of a protein of interest, and the first nucleic acid sequence is operatively linked with a second nucleic acid sequence comprising a 35S, or a plastocyanin, promoter.

Disclosed is a nucleic acid having a nucleotide sequence comprising nucleotides 1-1641 of SEQ ID NO:26 (GNT1-GnT-III), or comprising a nucleotide sequence that exhibits from about 80% to 100% identity with the nucleotides 1-1641 of SEQ ID NO:26, as determined using the following parameters: Program: blastn; Database: nr; Expect 10; filter: low complexity; Alignment: pairwise; Word size: 11, wherein the nucleotide sequence encodes a protein that modifies glycosylation of a protein of interest.

Also disclosed is a nucleic acid having a nucleotide sequence comprising a first nucleic acid sequence comprising nucleotides 1-1460 of SEQ ID NO:16 (GnT-III), or comprising a sequence that is from about 80% to 100% similar with the nucleotides 1-1460 of SEQ ID NO:16, as determined using the following parameters: Program: blastn; Database: nr; Expect 10; filter: low complexity; Alignment: pairwise; Word size: 11, wherein the first nucleic acid sequence encodes a protein that modifies glycosylation of a protein of interest, the first nucleic acid sequence operatively linked with a second nucleic acid sequence comprising a 35S or a plastocyanin promoter.

There is also provided a plant, a plant cell, or a seed comprising the nucleotide sequence of (A) as described above.

The present invention also pertains to a hybrid protein GNT1-GalT, comprising a CTS domain of N-acetylglucosaminyl transferase fused to a catalytic domain of beta-1,4galactosyltransferase, and comprising sequence SEQ ID NO:18. The amino acid sequence of SEQ ID NO:18 may be encoded by the nucleotide sequence SEQ ID NO:17. There is also provided a plant, a plant cell, or a seed comprising the hybrid protein as just described. There is also provided a plant, a plant cell, or a seed comprising the nucleic acid comprising the nucleotide sequence SEQ ID NO:17.

Disclosed is a hybrid protein GNT1-GnT-III, comprising a CTS domain of N-acetylglucosaminyl transferase fused to a catalytic domain of N-acetylglucosaminyltransferase III, and comprising the amino acid sequence SEQ ID NO:20. The amino acid sequence of SEQ ID NO:21 may be encoded by the nucleotide sequence SEQ ID NO:26. There is also provided a plant, a plant cell, or a seed comprising the hybrid protein as just described. There is also provided a plant, a plant cell, or a seed comprising the nucleic acid comprising the nucleotide sequence SEQ ID NO:26.

According to the present invention there is provided a method (1) for synthesizing a protein of interest comprising expressing within a plant or a portion of a plant, a nucleotide sequence encoding a first nucleotide sequence encoding a hybrid protein, GNT1-GalT, comprising a CTS domain of N-acetylglucosaminyl transferase (GNT1) fused to a catalytic domain of beta-1,4galactosyltransferase (GalT), the first nucleotide sequence operatively linked with a first regulatory region that is active in the plant, and a second nucleotide sequence for encoding the protein of interest, the second nucleotide sequence operatively linked with a second regulatory region that is active in the plant, and expressing the first and second nucleotide sequences to synthesize a protein of interest comprising glycans with modified N-glycosylation.

The first nucleotide sequence and the second nucleotide sequence as described above, are transiently expressed in the plant. Furthermore, the first regulatory region is may be a first tissue-specific promoter, and the second regulatory region is a second tissue-specific promoter. Each of the first and second tissue-specific promoters may be a plastocycanin promoter.

The present invention also provides a method (2) for synthesizing a protein of interest. The method is as described above (method 1), wherein the protein of interest may be an antibody. If the protein of interest is an antibody, then the second nucleotide sequence encoding the protein of interest comprises a nucleotide sequence 2A, operatively linked with a regulatory region 2A that is active in the plant, and a nucleotide sequence 2B, operatively linked with a regulatory region 2B that is active in the plant, and the product encoded by each of 2A and 2B combine to produce the antibody. The regulatory region 2A may be a plastocycanin promoter, and the regulatory region 2B may be a plastocycanin promoter.

The present invention also provides a method (1) or (2) as described above, wherein a third nucleotide sequence is expressed within the plant, the third nucleotide sequence encoding a suppressor of silencing is operatively linked with a third regulatory region that is active in the plant. The third nucleotide sequence encoding a suppressor of silencing may be, for example HcPro, TEV -p1/HC-Pro, BYV-p21, TBSV p19, TCV-CP, CMV-2b, PVX-p25, PVM-p11, PVS-p11, BScV-p16, CTV-p23, GLRaV-2 p24, GBV-p14, HLV-p10, GCLV-p16 or GVA-p10. The third tissue-specific promoter may be a plastocycanin promoter.

Disclosed is a plant expression system for driving the expression of a protein of interest in a plant, wherein the protein of interest comprises a modified glycosylation pattern. For example the protein of interest may comprise reduced fucosylated, xylosylated, or both, fucosylated and xylosylated, N-glycans. Alternatively, the protein of interest may comprise a modified glycosylation pattern, wherein the protein lacks fucosylated, xylosylated, or both fucosylated and xylosylated residues, and exhibits increased galatosylation. Furthermore, a terminal galactose may be added that results in a reduction or elimination of fucosylation and xylosylation of the protein of interest when compared to the same protein of interest produced in a wild-type plant.

Disclosed is a method (3) for synthesizing a protein of interest with a modified N-glycosylation profile comprising, co-expressing within a plant, a portion of a plant, or a plant cell, a nucleotide sequence encoding a first nucleotide sequence encoding a hybrid protein, GNT1-GnT-III, comprising a CTS domain of N-acetylglucosaminyl transferase (GNT1) fused to a catalytic domain of N-acetylglucosaminyltransferase III (GnT-III), the first nucleotide sequence operatively linked with a first regulatory region that is active in the plant, and a second nucleotide sequence for encoding the protein of interest, the second nucleotide sequence operatively linked with a second regulatory region that is active in the plant, and co-expressing the first and second nucleotide sequences to synthesize a protein of interest comprising glycans with the modified N-glycosylation profile.

The first nucleotide sequence and the second nucleotide sequence as described above (method 3), may be transiently expressed in the plant, or they may be stably expressed. Furthermore, the first regulatory region is may be a first tissue-specific promoter, and the second regulatory region is a second tissue-specific promoter. Each of the first and second tissue-specific promoters may be a plastocycanin promoter.

Also disclosed is a method (4) for synthesizing a protein of interest with a modified N-glycosylation profile comprising, co-expressing within a plant, a portion of a plant, or a plant cell, a nucleotide sequence encoding a first nucleotide sequence encoding a hybrid protein, GNT1-GalT, comprising a CTS domain of N-acetylglucosaminyl transferase (GNT1) fused to a catalytic domain of beta-1,4galactosyltransferase (GalT), the first nucleotide sequence operatively linked with a first regulatory region that is active in the plant, a second nucleotide sequence encoding beta-1,4galactosyltransferase, the second nucleotide sequence operatively linked with a second regulatory region that is active in the plant, and a third nucleotide sequence for encoding the protein of interest, the third nucleotide sequence operatively linked with a third regulatory region that is active in the plant, and co-expressing the first, second and third nucleotide sequences to synthesize a protein of interest comprising glycans with the modified N-glycosylation profile.

The first nucleotide sequence and the second nucleotide sequence as described above (4), may be transiently expressed in the plant, or they may be stably expressed. Furthermore, the first regulatory region is may be a first tissue-specific promoter, and the second regulatory region is a second tissue-specific promoter. Each of the first and second tissue-specific promoters may be a plastocycanin promoter.

Disclosed is a method (5) for synthesizing a protein of interest with a modified N-glycosylation profile comprising, co-expressing within a plant, a portion of a plant, or a plant cell, a nucleotide sequence encoding a first nucleotide sequence encoding a hybrid protein, GNT1-GnT-III, comprising a CTS domain of N-acetylglucosaminyl transferase (GNT1) fused to a catalytic domain of N-acetylglucosaminyltransferase III (GnT-III), the first nucleotide sequence operatively linked with a first regulatory region that is active in the plant, a second nucleotide sequence encoding N-acetylglucosaminyltransferase III, the second nucleotide sequence operatively linked with a second regulatory region that is active in the plant, and a third nucleotide sequence for encoding the protein of interest, the third nucleotide sequence operatively linked with a third regulatory region that is active in the plant, and co-expressing the first, second and third nucleotide sequences to synthesize a protein of interest comprising glycans with the modified N-glycosylation profile.

The first nucleotide sequence, the second nucleotide sequence and the third nucleotide sequence as described in the method (5) above, may be transiently expressed in the plant, or they may be stably expressed. Furthermore, the first, second and third regulatory region is may be tissue-specific promoters. For example, each of the tissue-specific promoters may be a plastocycanin promoter.

According to the methods described herein a protein of interest may therefore be produced in high yield and that lack glycans that are known to be involved in hypersensitivity reactions, or be otherwise involved in allergenic reactions. This is accomplished by co-expressing glyco-engineered enzymes along with the protein of interest, and this result in the production of a less immunogenic protein than would be produced within the wild-type plant.

As described herein, a simplified expression systems for the production of a protein of interest using a transient expression system may be used, however, the methods may also be used with stable transformation systems. The present invention is therefore not limited to transient expression systems.

By using transient co-expression, the system described herein avoids the lengthy production times, and the selection process of elite mutant or glyco-engineered transgenic lines and their subsequent use as parental lines (e.g. as described by Bakker, 2005). It also avoids the concurrent problems often encountered with mutant or glyco-engineered plants, in terms of productivity, pollen production, seed set (Bakker et al 2005) and viability (Boisson et al., 2005). As described herein, co-expression of a protein of interest with a modifying chimeric human galactosyltransferase had no effect on production kinetics or yields.

The transient expression system described herein yields expression levels reaching 1.5 g of high quality antibody per kilogram of leaf fresh weight, exceeding the accumulation level reported for any antibody in plants with other expression systems, including multi-virus based systems and transgenic plants.

The approach described herein, for example involving expression of GalT or GalT-GNT1, may also be used with stably transformed plants. While exhibiting many advantages described above, this invention is not limited to transient expression systems.

This summary of the invention does not necessarily describe all features of the invention.

### BRIEF DESCRIPTION OF THE DRAWINGS

These and other features of the invention will become more apparent from the following description in which reference is made to the appended drawings wherein:

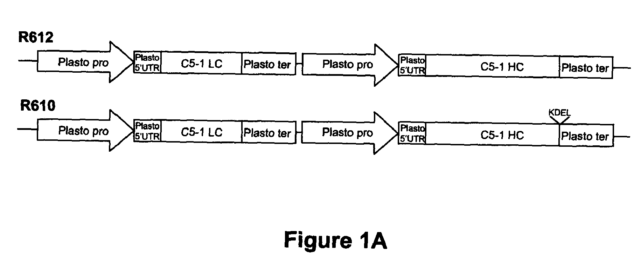

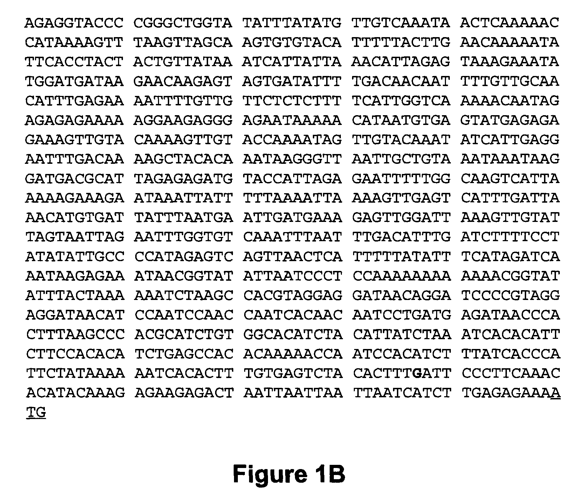

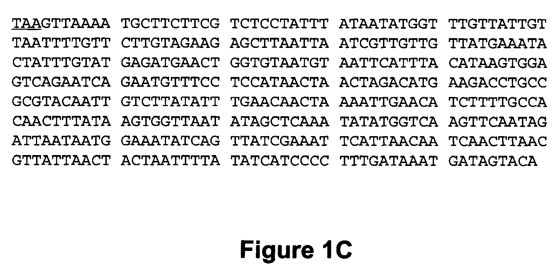

Figure 1A shows a plastocyanin-based cassette assembled for C5-1 expression. R610 comprises a nucleotide sequence encoding C5-1 LC and C5-1 HC-KDEL; R612 comprises a nucleotide sequence encoding C5-1 LC and C5-1 HC. C5-1 LC: C5-1 light chain coding sequence; C5-1 HC: C5-1 heavy chain coding sequence. Figure 1B shows the nucleotide sequence for the plastocyanin promoter and 5' UTR (SEQ ID NO:23), the transcription start site is shown in bold, and the translation start codon is underlined. Figure 1C shows the nucleotide sequence for the plastocyanin 3' UTR and terminator (SEQ ID NO:24), the stop codon is underlined.

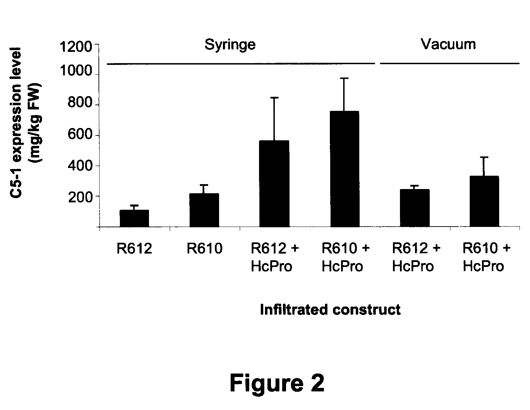

Figure 2 shows accumulation of the C5-1 antibody in leaves of *Nicotiana benthamiana* infiltrated with R610 and R612 (plastocyanin based expression cassettes) with or without co-expression of suppressor of silencing HcPro. The values presented correspond to the mean accumulation level and standard deviation obtained from the 6 measurements on 3 plants (syringe) or 6 measurements on individual infiltration batches of approximately 12 plants (250 g).

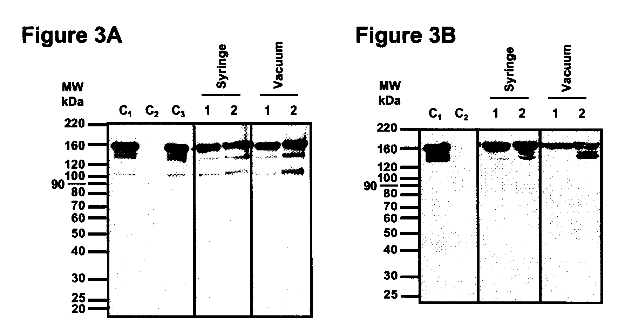

Figure 3 shows protein blot analysis of C5-1 accumulation in extracts of syringe- and vacuum-infiltrated plants. Figure 3A shows immunoblotting with a peroxidase-conjugated goat-anti mouse IgG (H+L), on extracts from plants infiltrated with R612 (for secretion, lanes 1) or with R610 (for ER-retention, lanes 2). C₁: 100 ng of commercial murine IgG1 (Sigma M9269), loaded as a control for electrophoretic mobility; C₂: 12 µg of total proteins extracted from mock-infiltrated biomass (empty vector). C₃: 100 ng of commercial murine IgG1 (Sigma M9269) spiked in 12 µg of total protein extracted from mock-infiltrated biomass (empty vector). Figure 3B shows activity immunoblotting with a peroxidase conjugated human IgG1, on extracts from plants infiltrated with R612 (for secretion, lanes 1) or with R610 (for ER-retention, lanes 2). C₁: 2µg of control C5-1 purified from hybridoma (Khoudi et al., 1997); C₂: 75 µg of total proteins extracted from mock-infiltrated biomass (empty vector).

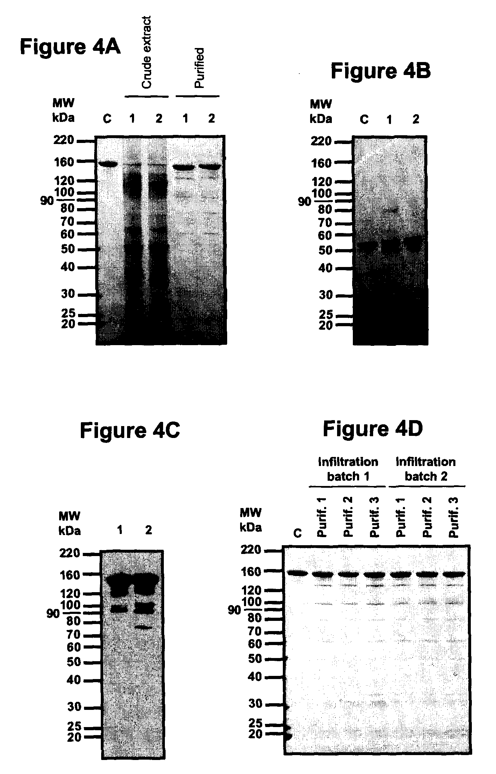

Figure 4 shows an analysis of antibodies purified from plants infiltrated with either R612 (for secretion, lanes 1) or R610 (for ER-retention, lanes 2). Figure 4A shows SDS-PAGE of crude extracts and purified antibodies performed in nonreducing conditions. Figure 4B shows SDS-PAGE of purified antibodies performed under reducing conditions Figure 4C shows activity immunoblotting of purified antibodies performed with a peroxidase conjugated human IgG1. Figure 4D shows a comparison of contaminants in 6 lots of purified C5-1 from different infiltration batches. C: 2.5 µg of commercial murine IgG1 (Sigma M9269), loaded as a control for electrophoretic mobility.

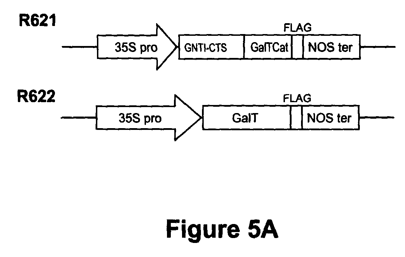

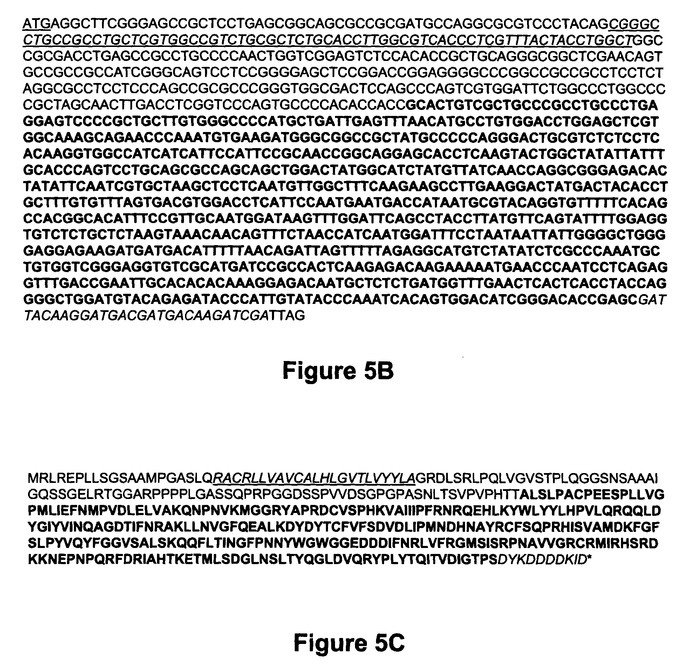

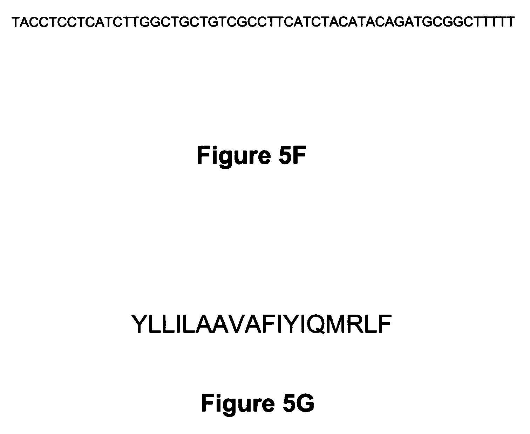

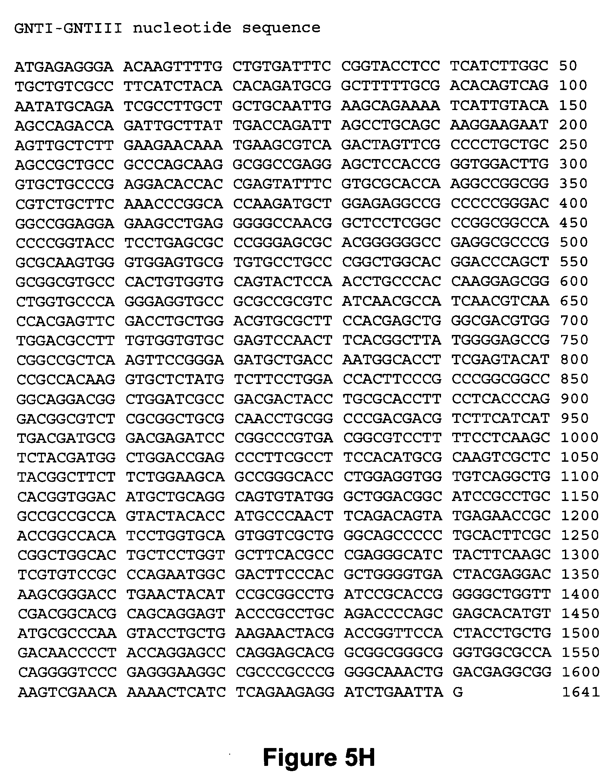

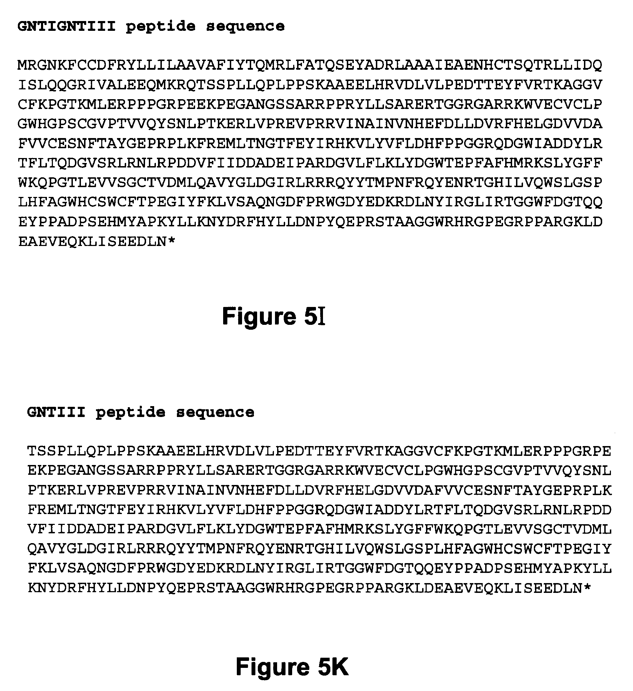

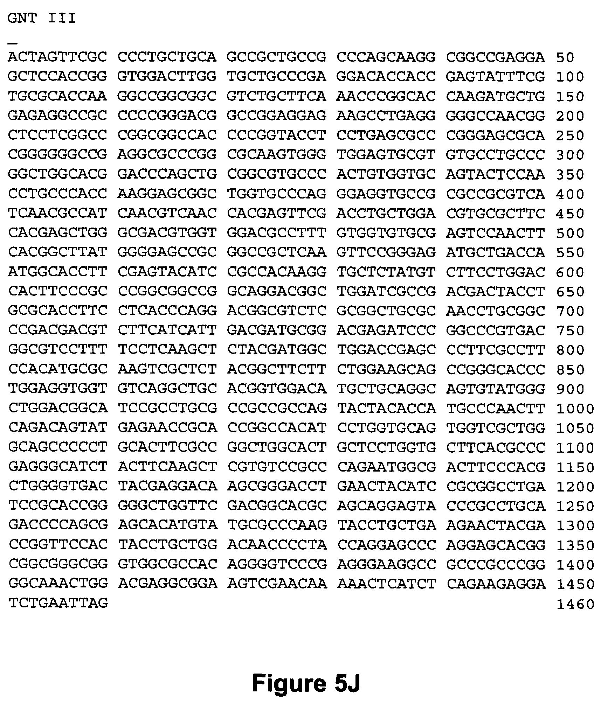

Figure 5A shows a representation of examples of cassettes assembled for native (R622) and hybrid (R621) versions of galactosyltransferase expression. GNTI-CTS: CTS domain of N-acetylglucos-aminyltransferase I; GalT-Cat: catalytic domain of human β1,4galactosyltransferase; GalT (of R622): human β1,4galactosyltransferase. Figure 5B shows the nucleotide sequence (SEQ ID NO: 14) for GalT (UDP-Gal:betaGlcNac beta 1,4-galactosyltransferase polypeptide 1, beta-1,4-galactosyltrasnferase I), the ATG start site is underlined; the transmembrane domain is underlined and in italics; the sequence in bold corresponds to the catalytic domain of human beta1,4GalT; the FLAG epitope is in italics. Figure 5C shows the amino acid sequence (SEQ ID NO: 15) for GalT (UDP-Gal:betaGlcNac beta 1,4-galactosyltransferase polypeptide 1, beta-1,4-galactosyltrasnferase I). The transmembrane domain is underlined and in italics; the sequence in bold corresponds to the catalytic domain of human beta1,4GalT; the FLAG epitope is in italics. Figure 5D shows the nucleotide sequence (SEQ ID NO: 17) of GNTIGalT, the ATG start site is underlined; the transmembrane domain (CTS) is underlined and in italics; the sequence in bold corresponds to the catalytic domain of human beta1,4GalT; the FLAG epitope is in italics. Figure 5E shows the amino acid sequence (SEQ ID NO: 18) of GNTIGalT. the transmembrane domain (CTS) is underlined and in italics; the sequence in bold corresponds to the catalytic domain of human beta1,4GalT; the FLAG epitope is in italics. Figure 5F shows the nucleotide sequence of a CTS domain (cytoplasmic tail, transmembrane domain, stem region) of N-acetylglucosamine transferase (GNT1; SEQ ID NO:21). Figure 5G shows the amino acid of the CTS (SEQ ID NO:22). Figure 5H shows the nucleic acid sequence of GntI-Gnt III (SEQ ID NO:26). Figure 5I shows the amino acid sequence of GntI-Gnt III (SEQ ID NO:20). Figure 5J shows the nucleic acid sequence of Gnt III (SEQ ID NO:16). Figure 5K shows the amino acid sequence of Gnt III (SEQ ID NO:19).

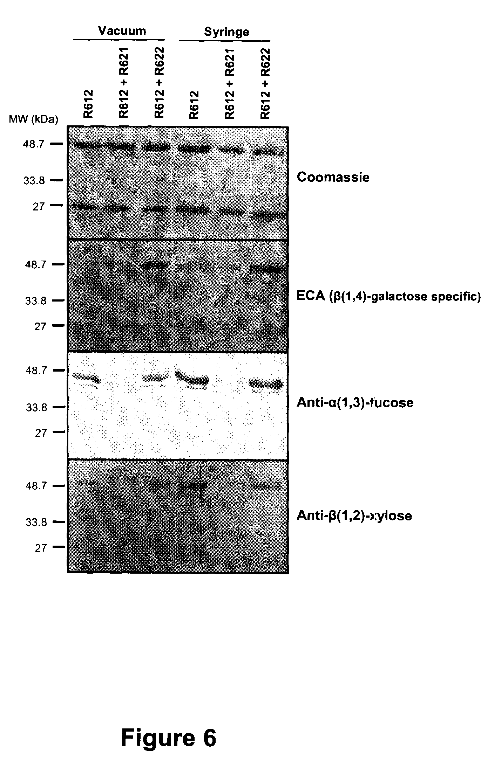

Figure 6 shows a profile of extracts obtained from plants expressing C5-1 and either stained for protein, or subject to Western analysis. Top panel shows a Coomasie stained PAGE gel. Second from the top panel shows affinodetection using *Erythrina cristagali* agglutinin (ECA) which specifically binds β1,4galactose. Third panel from the top shows Western blot analysis using anti-α1,3fucose antibodies. Bottom panel shows Western blot analysis using anti-β1,2xylose specific antibodies. R612: C5-1 expressed alone; R612+R622: C5-1 co-expressed (co-infiltrated) with GalT; R612+R621: C5-1 co-expressed with GNT1-GalT.

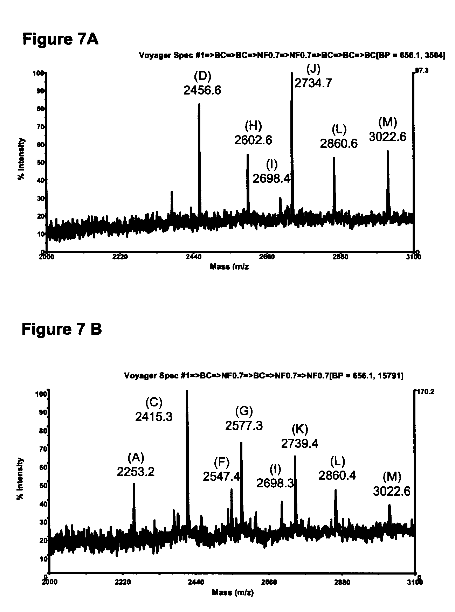

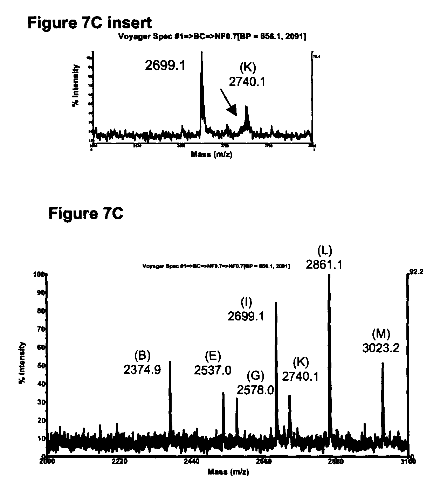

Figure 7 shows MALDI-TOF mass spectrometry to determine N-glycosylation of the tryptic glycopeptide EEQFNSTFR (SEQ ID NO:13) of C5-1 isolated from *Nicotiana benthamiana* syringe-infiltrated plants with a range of constructs. N-glycosylation of the glycopeptide was determined after separation on preparative HPLC. Similar results were obtained following vacuum infiltration. Figure 7A shows MALDI-TOF mass spectrometry of the tryptic glycopeptide following expression of R612 (C5-1; see Figure 1). Figure 7B shows MALDI-TOF mass spectrometry of the tryptic glycopeptide following expression of C5-1 (R612, see Figure 1) along with native GalT (R622, see Figure 5). Figure 7C shows MALDI-TOF mass spectrometry of the tryptic glycopeptide following expression of C5-1 (R612, see Figure 1;) with GNTIGalT (R621, see Figure 5). Figure 7C-insert, corresponds to the m/z 2650-2800 enlargement of the spectrum to show the absence of the main complex ion J detected in the R612 plant (arrow). A: GlcNAcMan₃GlcNAc,; B: Man₅GlcNAc; C: GalGlcNAcMan₃GlcNAc; D: GlcNAc₂Man₃GlcNAc,; E: Man₆GlcNAc₂; F: GalGlcNAcMan₃(Xyl)GlcNAc₂; G: GlcNAcMan₅GlcNAc₂; H: GlcNAc₂Man₃(Fuc)GlcNAc₂; I: Man₇GlcNAc₂; J: GlcNAc₂Man₃(Xyl)(Fuc)GlcNAc; K: GalGlcNAcMan₅GlcNAc; L: Man₈GlcNAc₂; M: Man₉GlcNAc₂.

### DETAILED DESCRIPTION

The present invention relates to methods for modifying glycoprotein production in plants. The present invention also provides plants with modified glycoprotein production.

Disclosed is a plant expression system for driving the expression of a protein of interest in a plant. With the expression system described, a protein of interest comprising a modified glycosylation pattern, for example with reduced fucosylated, xylosylated, or both, fucosylated and xylosylated, N-glycans may be obtained. Alternatively, a protein of interest comprising a modified glycosylation pattern may be obtained, wherein the protein lacks fucosylation, xylosylation, or both, and comprises increased galatosylation. Furthermore, as described herein, modulation of post-translational modifications, for example, the addition of terminal galactose results in a reduction of fucosylation and xylosylation of the expressed protein of interest, for example the protein of interest may comprise less than 10% fucosylation and xylosylation (i.e. less than 10% of the N-gylcan residues are fucoylated and xylosyated), or less than 5% fucosylation and xylosylation (i.e. less than 5% of the N-gylcan residues are fucoylated and xylosyated), less than 1% fucosylation and xylosylation (less than 1% of the N-gylcan residues are fucoylated and xylosyated), from about 0.1 to about 2% of the N-glycan residues are fucoylated and xylosyated, from about 0.5 to about 1.5% of the N-glycan residues are fucoylated and xylosyated, or from about 0.7 to about 1.0 % of the N-glycan residues are fucoylated and xylosyated, when compared to the same protein of interest produced in a wild-type plant. A protein of interest may therefore be produced in high yield and lack glycans that may provoke hypersensitivity reactions, or be otherwise involved in allergenic reactions.

A non-limiting example of a protein of interest to be expressed includes a complex protein such as an antibody. Expression of such a complex protein within an agroinfiltrated plant, for example *Nicotiana benthamiana,* produced levels of protein reaching 1.5 g/kg FW (approx. 25% TSP). Average levels of 558 and 757 mg/kg/FW were attained for the secreted and ER-retained forms of the protein of interest, respectively. In the non-limiting example provided, this expression level was obtained for an antibody, at level of expression threefold higher than for an antibody produced using a multi-virus transient expression system (Giritch et al. 2006).

The impact of the difference between plant and typical mammalian N-glycosylation has been a major concern surrounding the concept of using plants for therapeutics production. The occurrence of plant-specific glycans may contribute to shorten the half-life of a plant-made protein in the blood stream, or that the same glycans provoke hypersensitivity reactions in patients.

The presence of core α1,3fucose and β1,2xylose on glyco-proteins made in plants is perceived as a regulatory challenge by the industry as they are also found on some plant allergens. In addition, it is now documented that the removal of core fucose, even α1,6fucose which would be found in IgGs from CHO cells, will increase ADCC activity. A feature of the system described herein is the capacity to accomplish modulation of post-translational modifications, for example, the concomitant addition of terminal galactose and reduction or inhibition of fucosylation and xylosylation (when compared to the same protein produced in a wild-type plant). Alternatively, the degree of fucsolylation may be reduced while increasing the amount of galatosylation, again when compared to the same protein produced in a wild-type plant.

The modulation in the amount of fucosylation, xylosylation or galatosylation, may be determined using any suitable method, for example using anti-alpha-1.3fucose antibodies, to detect the presence or absence of fucose-specific immunosignals (fucosylation), and anti-beta1,2xylose antibodies to detect xylosylation, or the presence or absence of xylose-specific immunosignals, for example, as shown in Figure 6. Alternatively, MALDI-TOF mass spectrometry may be used to determine the N glycosylation profile of a protein or a portion of the protein, as shown in Figure 7. Other method to determine the N-glycan profile of a protein or portion of the protein known to one of skill in the art may also be used.

As described in more detail below, a vacuum-based agroinfiltration system was used and found suitable for the production of a protein of interest, for example an antibody in terms of quantity, quality and reproducibility. The passage to a scalable infiltration technology allows for the production of grams of this antibody per day within a small pilot unit, which permits the use of such transient expression system for the production of materials for clinical trials within extremely short time frames and for the supply of a licensed product with a market size up to kilograms per year. High quality antibodies were obtained from infiltrated leaves after a single affinity chromatographic step. However, it is to be understood that the methods described herein may also apply to plants that are stably transformed.

Post-transcriptional gene silencing (PTGS) may be involved in limiting expression of transgenes in plants, and co-expression of a suppressor of silencing, for example, but not limited to HcPro, from the potato virus Y may be used to counteract the specific degradation of transgene mRNAs (Brigneti et al., 1998). Alternate suppressors of silencing are well known in the art and may be used as described herein (Chiba et al., 2006, Virology 346:7-14), for example but not limited to TEV -p1/HCPro (Tobacco etch virus-p1/HC-Pro), BYV -p21, p19 of Tomato bushy stunt virus (TBSV p19), capsid protein of Tomato crinkle virus (TCV -CP), 2b of Cucumber mosaic virus; CMV-2b), p25 of Potato virus X (PVX-p25), p11 of Potato virus M (PVM-p11), p11 of Potato virus S (PVS-p11), p16 of Blueberry scorch virus, (BScV-p16), p23 of Citrus tristexa virus (CTV-p23), p24 of Grapevine leafroll-associated virus-2, (GLRaV-2 p24), p10 of Grapevine virus A, (GVA-p10), p14 of Grapevine virus B (GVB-p14), p10 of Heracleum latent virus (HLV-p10), or p16 of Garlic common latent virus (GCLV-p16). Therefore, a suppressor of silencing, for example HcPro, TEV -p1/HC-Pro, BYV-p21, TBSV p19, TCV-CP, CMV-2b, PVX-p25, PVM-p11, PVS-p11, BScV-p16, CTV-p23, GLRaV-2 p24, GBV-p14, HLV-p10, GCLV-p16or GVA-p10, may be co-expressed along with either GalT, GNT1-GalT, GnT-III, GNT1-GnT-III, or a combination thereof, to further ensure high levels of protein production within a plant.

The Coomassie staining of purified products produced using transient expression shows the presence of various contaminants of low abundance. These fragments appear to be product related, and all contaminants over 70 kDa contained at least one Fab as shown by the activity blot (Figure 3B). The identity and quantity of product related contaminants present in plant extracts being similar to those observed in mammalian cell production systems. Therefore, a purification train typically used for the purification of therapeutic antibodies (e.g. anion exchange, affinity and cation exchange) easily yields the purity required by the regulatory agencies for a protein for therapeutic use.

The present invention provides a method for the synthesis of a protein of interest within plants that are characterized as having a modified glycosylation pattern. The method involves co-expressing the protein of interest along with a nucleotide sequence encoding beta-1.4galactosyltransferase (GalT; SEQ ID NO:14), for example, but not limited to mammalian GalT, or human GalT however GalT from another sources may also be used. The catalytic domain of GalT (for example nucleotides 370- 1194 of SEQ ID NO:14, bold sequence in Figure 5b, or nucleotides 238- 1062 of SEQ ID NO: 17, bold sequence in Figure 5d) **are** fused to a CTS domain (i.e. the cytoplasmic tail, transmembrane domain, stem region) of N-acetylglucosaminyl transferase (GNT1; for example, comprising nucleotides 34-87 of SEQ ID NO:17; Figure 5d) and encoding the amino acid sequence comprising amino acids 12-29 of SEQ ID NO: 18 (Figure 5e), to produce a GNT1-GalT hybrid enzyme, and the hybrid enzyme may be co-expressed with the protein of interest. Disclosed isthe co-expression of the protein of interest along with a nucleotide sequence encoding N-acetylglucosaminyltrasnferase III (GnT-III; SEQ ID NO:16; Figure 5j), for example but not limited to mammalian GnT-III or human GnT-III, GnT-III from other sources may also be used. Additionally, a GNT1-GnT-III hybrid enzyme (SEQ ID NO:26; Figure 5d), comprising the CTS of GNT1 fused to GnT-III may also be used and is described below.

Alternate methods for anchoring the nucleotide sequence encoding GalT mGalT, or hGalT or GalT from other sources, include fusing the GalT to HDEL, KDEL (both endoplasmic reticulum retention sequences), the CTS of a protein involved in N-glycoprotein biosynthesis, for example but not limited to the CTS of glucosidase I, CTS of glucosidase II, CTS of mannosidase I, CTS of mannosidase II, CTS of beta,1,2xylsyltrasnferease, CTS of alpha, 1,2fucosyltrasnferase. The catalytic domain of GalT may also be fused to HDEL, KDEL (both endoplasmic reticulum retention sequences), the CTS of glucosidase I, CTS of glucosidase II, CTS of mannosidase I, CTS of mannosidase II, CTS of beta,1,2xylsyltrasnferease, CTS of alpha, 1,2fucosyltrasnferase.

The use of a hybrid enzyme either comprising the GNT1-GalT, or the GNT1-GnT-III sequence, positions the catalytic domain of GalT, or GnT-III, in the cis-Golgi apparatus where early stages in complex N-glycan maturation occurs. Without wishing to be bound by theory, sequestering GalT activity at an early stage of glycan maturation may result in the addition of β1,4galactose on maturating glycans and result in the efficient inhibition of fucosylation and xylosylation of the protein that would otherwise take place within the plant. Similarly, sequestering GnT-III activity at an early stage of glycan maturation may result in the addition of GlcNAc residues on beta-linked mannose to produce a bisecting GlnAc, and result in the efficient inhibition of fucosylation and xylosylation of the protein that would otherwise take place within the plant. For example, the protein of interest may be co-expressed with a hybrid enzyme comprising a CTS domain fused to a GalT catalytic domain, for example GNT1-GalT (R621; Figures 5a, 5d), or a GnT-III catalytic domain, for example GNT1-GnT-III (SEQ ID NO:26; Figure 5h). If a protein of interest comprising reduced levels of fucoslylation, while still comprising xylosylated and galactosylated proteins is desired, then unmodified (native) GalT may be co-expressed with the protein of interest. If a protein of interest comprising modified glycosylation comprising bisecting GlnAc residues is desired, then unmodified (native) GnT-III, may be co-expressed with the protein of interest. As one of skill in the art would appreciate, a plant optimized nucleic acid sequence may be used to produce unmodified or native GalT or GnT-III enzyme.

Therefore, there is provided a nucleotide sequence comprising nucleotides 1-1062 of SEQ ID NO:17 (GNT1-GalT), or comprising a nucleotide sequence that exhibits from about 80% to 100% identity with the nucleotides 1-102 of SEQ ID NO:17, wherein the nucleotide sequence encodes a protein that modifies glycosylation of a protein of interest. The sequence may be plant optimized. There is also disclosed a nucleotide sequence that comprises a nucleic acid sequence comprising nucleotides 1-1224 of SEQ ID NO:14 (GalT), or comprising a sequence that exhibits from about 80% to 100% identity with the nucleotides 1-1224 of SEQ ID NO:14, wherein the nucleic acid sequence encodes a protein that modifies glycosylation of a protein of interest. The sequence may be plant optimized. The sequence identity is determined using the following parameters: Program: blastn; Database: nr; Expect 10; filter: low complexity; Alignment: pairwise; Word size: 11.

There is also disclosed an amino acid sequence as set forth in SEQ ID NO:18 (GNT1-GalT; Figure 5e), or SEQ ID NO: 15 (GalT; Figure 5c).

There is disclosed a nucleic acid having nucleotide sequence comprising nucleotides 1-1641 of SEQ ID NO:26 (GNT1-GnT-III), or comprising a nucleotide sequence that exhibits from about 80% to 100% identity with the nucleotides 1-1641 of SEQ ID NO:26, wherein the nucleic acid sequence encodes a protein that modifies glycosylation of a protein of interest. The sequence may be plant optimized. There is also provided is a nucleotide sequence that comprises a nucleic acid sequence comprising nucleotides 232-1641 of SEQ ID NO:26 (GnT-III; or nucleotides 1-1460 of SEQ ID NO:16), or comprising a sequence that exhibits from about 80% to 100% identity with the nucleotides 232-1641 of SEQ ID NO:26 (or nucleotides 1-1460 of SEQ ID NO:16), wherein the nucleic acid sequence encodes a protein that modifies glycosylation of a protein of interest. The sequence may be plant optimized. The sequence identity is determined using the following parameters: Program: blastn; Database: nr; Expect 10; filter: low complexity; Alignment: pairwise; Word size: 11.wherein the nucleotide sequence encodes a protein that modifies glycosylation of a protein of interest.

There is also disclosed an amino acid sequence as set forth in SEQ ID NO:20 (GNT1-GnT-III; Figure 5i), or SEQ ID NO:19 (GnT-III, Figure 5k).

By "modified glycosylation" of a protein of interest it is meant that the N-glycan profile of the protein of interest comprising modified glycosylation (for example, as described herein), is different from that of the N-glycan profile of the protein of interest produced in a wild-type plant. Modification of glycosylation may include an increase or a decrease in one or more than one glycan of the protein of interest, or the bisecting of GlnAc. For example, the protein of interest may exhibit reduced xylosylation, reduced fucosylation, or both reduced xylosylation and reduced fucosylation. Alternatively, the N-glycan profile of the protein of interest may be modified in a manner so that the amount of galactosylation is increased, and optionally, the amount xylosylation, fucosylation, or both, are reduced. Additionally, bisected GlnAc may be produced and this may result in the reduction of the amount of fucosylation and xylosylation of the protein.

By "reduced xylosylation", or "reduced fucosylation" of a protein of interest, it is meant that the amount of xylosylation, fucosylation, or both xylosylation and fucosylation, of N-glycans detectable on the protein of interest is less than 10% than the amount xylosylation, fucosylation, or both, that is detectable on the protein of interest when produced within a wild-type plant, and where the protein of interest is isolated, and where xylosylation or fucosylation is determined, using the same method. For example, the protein of interest may comprise less than 5% of the N-glycan residues that are fucoylated, xylosyated or both, less than 1% of the N-gylcan residues detectable on the protein of interest may be fucoylated, xylosyated, or both, from about 0.1 to about 2% of the N-glycan residues that are detecable on the protein of interest may be fucoylated and xylosyated, from about 0.5 to about 1.5% of the N-glycan residues are fucoylated and xylosyated, or from about 0.7 to about 1.0 % of the N-glycan residues are fucoylated and xylosyated, when compared to the same protein of interest produced in a wild-type plant.

As shown in Figures 6 and 7, by using the methods described herein, a protein of interest may be produced that exhibits a modified glycosylation profile. For example, a protein of interest with immunologically undetectable fucose or xylose residues has been produced when the protein of interest is co-expressed with GNT1-GalT. MALDI-TOF analysis of an epitope of a protein of interest demonstrates that a protein of interest with a modified glycosylation pattern may be obtained when the protein of interest is co-expressed with either GalT or GNT1-GalT (see Figures 7A-C, C-insert). For example Figure 7A, shows the glycosylation profile of an epitope of a protein of interest expressed within a wild-type plant. The protein of interest comprises several xylosylated and fucosylated residues (peaks H and J, respectively, Figure 7A). These residues are reduced or absent when the protein of interest is co-expressed with GalT (Figure 7B). Furthermore, new xylosylated residues are observed (peak F), as well as an increase in galactosylated residues in the protein of interest, when co-expressed with GalT (see peaks C, F K, Figure 7B). Co-expression of the protein of interest with GNT1-GalT results in a N-glycan profile that is characterized as having less than 1% xylosylated and fucosylated residues (see Figure 7C-insert), and an increase in galactosylated residues (peak K; Figure 7C).

Therefore, disclosed is a method (Method A) of synthesizing a protein of interest with modified N-glycosylation comprising, providing a plant comprising a nucleotide sequence encoding a first nucleotide sequence encoding human beta-1,4galactosyltransferase (GalT), the first nucleotide sequence operatively linked with a regulatory region that is active in the plant, and a second nucleotide sequence for encoding the protein of interest, the second nucleotide sequence operatively linked with a regulatory region that is active in the plant, growing the plant, and expressing the first and second nucleotide sequences to synthesize a protein of interest comprising modified N-glycosylation. The co-expression of the first sequence encoding GalT along with the second sequence encoding the protein of interest, results in the addition of beta-1,4 galactose on maturing glycans of the protein of interest, thereby reducing fucoslylation, xylosylation, or both, of the glycans of the protein of interest. Furthermore, with this method the degree of galactosylation of the protein of interest may be increased when compared to the amount of galactosylation of the protein of interest produced in a wild-type plant that does not express GalT.

A method is disclosed (Method B) for synthesizing a protein of interest with modified N-glycosylation that involves expressing within a plant or a portion of a plant, in a transient manner, a nucleotide sequence encoding a first nucleotide sequence encoding human beta-1,4galactosyltransferase (GalT; SEQ ID NO:14; Figure 5b), the first nucleotide sequence operatively linked with a regulatory region that is active in the plant, and a second nucleotide sequence for encoding the protein of interest, the second nucleotide sequence operatively linked with a regulatory region that is active in the plant, and expressing the first and second nucleotide sequences to synthesize a protein of interest comprising glycans with modified N-glycosylation. Co-expression of the first sequence encoding GalT along with the second sequence encoding the protein of interest, results in the addition of beta-1,4 galactose on maturing glycans of the protein of interest, thereby reducing fucoslylation, xylosylation, or both, of the glycans of the protein of interest. Furthermore, the degree of galactosylation of the protein of interest may be increased when compared to the amount of galactosylation of the protein of interest produced in a wild-type plant that does not express GalT. The step of expressing may involve transiently co-expressing the first and second nucleotide sequence, or stably co-expressing the first and second nucleotide sequence.

The present invention provides a method (Method C) of synthesizing a protein of interest with modified N-glycosylation comprising, providing a plant comprising a first hybrid nucleotide sequence encoding a first hybrid protein comprising a CTS domain of N-acetylglucosaminyl transferase (GNT1) fused to the catalytic domain of GalT (human beta-1.4galactosyltransferase; GNT1-GalT; SEQ NO:17; Figure 5d), the first hybrid nucleotide sequence operatively linked with a regulatory region that is active in the plant, and a second nucleotide sequence for encoding the protein of interest, the second nucleotide sequence operatively linked with a regulatory region that is active in the plant, growing the plant, and transiently expressing the first hybrid, and second, nucleotide sequences to synthesize a protein of interest comprising modified N-glycosylation. Co-expression of the first hybrid sequence encoding GNT1-GalT along with the second sequence encoding the protein of interest results in the addition of beta-1,4 galactose on maturing glycans of the protein of interest, thereby reducing the fucosylation and xylosylation of the glycans of the protein of interest. For example, the degree of fucosylation, xylosylation, or both, may be reduced from about 0.5 to about 5%, or from about 0.5 to about 2%, of the amount when compared to the protein of interest produced in a wild-type plant that does not express GNT1-GalT.

An additional alternate method is disclosed (Method D) for synthesizing a protein of interest with modified N-glycosylation that involves expressing within a plant or a portion of a plant, in a transient manner, a nucleotide sequence encoding a first hybrid nucleotide sequence encoding GNT1-GalT, the first hybrid nucleotide sequence operatively linked with a regulatory region that is active in the plant, and a second nucleotide sequence for encoding the protein of interest, the second nucleotide sequence operatively linked with a regulatory region that is active in the plant, and expressing the first and second nucleotide sequences to synthesize a protein of interest comprising modified N-glycosylation. Co-expression of the first hybrid sequence encoding GNT1-GalT along with the second sequence encoding the protein of interest, results in the addition of beta-1,4 galactose on maturing glycans of the protein of interest, thereby reducing the fucosylation and xylosylation of the glycans of the protein of interest. For example, the degree of fucosylation, xylosylation, or both, may be reduced from about 0.5 to about 5%, or from about 0.5 to about 2%, of the amount when compared to the protein of interest produced in a wild-type plant that does not express GNT1-GalT. The step of expressing may involve transiently co-expressing the first and second nucleotide sequence, or stably co-expressing the first and second nucleotide sequence.

Disclosed is an additional alternate method (Method E) for synthesizing a protein of interest with modified N-glycosylation comprising, providing a plant comprising a nucleotide sequence encoding a first nucleotide sequence encoding human beta-1,4galactosyltransferase (GalT), the first nucleotide sequence operatively linked with a regulatory region that is active in the plant, a second nucleotide sequence for encoding the protein of interest, the second nucleotide sequence operatively linked with a regulatory region that is active in the plant, and a third nucleotide sequence encoding a suppressor of gene silencing, for example HcPro, the third nucleotide sequence operatively linked with a regulatory region that is active in the plant, growing the plant, and expressing the first, second, and third nucleotide sequences to synthesize a protein of interest comprising glycans with modified N-glycosylation. The co-expression of the first sequence encoding GalT along with the second sequence encoding the protein of interest, results in the addition of beta-1,4 galactose on maturing glycans of the protein of interest, thereby reducing the fucosylation and xylosylation of the glycans of the protein of interest, when compared with the protein of interest produced using a wild-type plant that does not express GalT. The degree of galactosylation of the protein of interest may be increased when compared to the amount of galactosylation of the protein of interest produced in a wild-type plant that does not express GalT. Expression of the third sequence encoding a suppressor of silencing, ensures high yields of the galactosyltransferase and the protein of interest.

There is also disclosed a method for synthesizing a protein of interest (Method F) with modified N-glycosylation that involves expressing within a plant or a portion of a plant, in a transient manner, a nucleotide sequence encoding a first nucleotide sequence encoding human beta-1,4galactosyltransferase (GalT), the first nucleotide sequence operatively linked with a regulatory region that is active in the plant, a second nucleotide sequence for encoding the protein of interest, the second nucleotide sequence operatively linked with a regulatory region that is active in the plant, and a third nucleotide sequence encoding a suppressor of gene silencing, for example HcPro, the third nucleotide sequence operatively linked with a regulatory region that is active in the plant, and expressing the first, second and third nucleotide sequences to synthesize a protein of interest comprising modified N-glycosylation. Co-expression of the first sequence encoding GalT along with the second sequence encoding the protein of interest, results in the addition of beta-1,4 galactose on maturing glycans of the protein of interest, thereby reducing the fucosylation and xylosylation of the glycans of the protein of interest. The degree of galactosylation of the protein of interest may be increased when compared to the amount of galactosylation of the protein of interest produced in a wild-type plant that does not express GalT. Expression of the third sequence encoding a suppressor of silencing, ensure high yields of the galactosyltransferase and the protein of interest. The step of expressing may involve transiently co-expressing the first and second nucleotide sequence, or stably co-expressing the first and second nucleotide sequence.

Therefore, disclosed is a method (Method G) of synthesizing a protein of interest with modified N-glycosylation comprising, providing a plant comprising a nucleotide sequence encoding a first nucleotide sequence encoding N-acetylglucosaminlyltransferase (GnT-III), the first nucleotide sequence operatively linked with a regulatory region that is active in the plant, and a second nucleotide sequence for encoding the protein of interest, the second nucleotide sequence operatively linked with a regulatory region that is active in the plant, growing the plant, and expressing the first and second nucleotide sequences to synthesize a protein of interest comprising modified N-glycosylation. The co-expression of the first sequence encoding GnT-III along with the second sequence encoding the protein of interest, results in the addition of beta-1,4-linked N-acetylglucosamine (GlnAc) residues to beta-linked mannose (bisecting GlnAc) on maturing glycans of the protein of interest, thereby reducing fucoslylation, xylosylation, or both, of the glycans of the protein of interest, when compared to the protein of interest produced in a wild-type plant that does not express GnT-III. The sequence encoding GnT-III (first nucleotide sequence), the protein of interest (second nucleotide sequence), or both the first and second nucleotide sequence may be transiently expressed. If the sequences are transiently expressed, a third nucleotide sequence encoding a suppressor of gene silencing, for example HcPro, operatively linked with a regulatory region active in the plant, may also be used and the first, second, and third nucleotide sequences expressed to synthesize a protein of interest comprising modified glycosylation.

Also disclosed is an alternate method (Method H) of synthesizing a protein of interest with modified N-glycosylation comprising, providing a plant comprising a first hybrid nucleotide sequence encoding a first hybrid protein encoding a CTS domain of N-acetylglucosaminyl transferase (GNT1) fused to the catalytic domain of GnT-III (N-acetylglucosaminlyltransferase; GNT1-GnT-III SEQ ID NO:20), the first hybrid nucleotide sequence operatively linked with a regulatory region that is active in the plant, and a second nucleotide sequence for encoding the protein of interest, the second nucleotide sequence operatively linked with a regulatory region that is active in the plant, growing the plant, and expressing the first hybrid, and second, nucleotide sequences to synthesize a protein of interest comprising modified N-glycosylation. Co-expression of the first hybrid sequence encoding GNT1-GnT-III along with the second sequence encoding the protein of interest results in the addition of beta-1,4-linked N-acetylglucosamine (GlnAc) residues to beta-linked mannose (bisecting GlnAc) on maturing glycans of the protein of interest maturing glycans of the protein of interest, thereby reducing the fucosylation and xylosylation of the glycans of the protein of interest. For example, the degree of fucosylation, xylosylation, or both, may be reduced from about 0.5 to about 5%, or from about 0.5 to about 2%, of the amount when compared to the protein of interest produced in a wild-type plant that does not express GNT1-GnT-III. The sequence encoding GNT1-GnT-III (first nucleotide sequence), the protein of interest (second nucleotide sequence), or both the first and second nucleotide sequence may be transiently expressed. If the sequences are transiently expressed, a third nucleotide sequence encoding a suppressor of gene silencing, for example HcPro, operatively linked with a regulatory region active in the plant, may also be used and the first, second, and third nucleotide sequences expressed to synthesize the protein of interest comprising modified glycosylation.

Additional modifications to the nucleotide sequence encoding the protein of interest may be made to ensure high yield. For example, the second sequence encoding the protein of interest may also be fused to a sequence encoding a sequence active in retaining the protein within the endoplasmic reticulum (ER), for example but not limited to, a KDEL (Lys-Asp-Glu-Leu ) sequence, or other known ER retention sequences for example HDEL, or KKSS.

Also, when complex protein of interest are produced, the second nucleotide sequence, used in any of Methods A-H as described above, may encode more than one peptide or domain of the complex protein. For example, in the case where the protein of interest is an antibody, the second nucleotide sequence may comprise two second nucleotide sequences, 2A and 2B, each encoding a portion of the antibody, for example nucleotide 2A may encode a light chain and a sequence 2B encodes a heavy chain of the antibody. Non-limiting examples of such constructs are provided in Figure 1, where construct each of R216 and R610 comprise two second nucleotide sequences 2A, encoding C5-1 LC (the light chain of C5-1) operatively linked to a regulatory region active in a plant, for example, but not limited to the plastocyanin promoter and 2B encoding the heavy chain of C5-1 (C5-1 HC) operatively linked to a regulatory region active in a plant, for example, but not limited to the plastocyanin promoter comprising nucleotides 556-999 of Figure 1b or SEQ NO:23; US 7,125,978). As shown in Figure 1, and with reference to R610, a KDEL sequence may be fused to the c-terminal region of one of the peptides 2A or 2B, for example which is not to be considered limiting, the KDEL sequence may be fused to the heavy chain of the antibody to ensure that the antibody is retained with the ER.

Each protein encoded by the second nucleotide sequence may be glycosylated.

The protein of interest so produced by any one of the Methods A-H of may be recovered from the plant. Furthermore, the protein of interest may be partially purified of purified using standard techniques as would be known to one of skill in the art.

In the cases where nucleotide sequences are co-expressed within the plant, each of the desired nucleotide sequences may be introduced into the plant using standard transformation techniques, transient transformation techniques, two or more than two plants, each expressing one or more of the desired nucleotide sequences may be crossed to obtain a plant that co-expresses the required combination of nucleotide sequences, or a combination of the above techniques may be combined. For example, transient expression may be carried out using a stably transformed plant expressing a sequence encoding GalT, GNT1-GalT, GalT and GNT1-GalT, GnT-III, GNT1-GnT-III, GNT1-Gnt-III and Gnt-III, or a combination thereof.

Therefore, disclosed is a method (Method I) for producing a plant that may be used as a platform for the production of a protein of interest with modified N-glycosylation. This method comprises, providing a plant that expresses one or more than one first nucleotide sequence encoding GalT, GNT1-GalT, or both GalT and GNT1-GalT, and expressing the one or more nucleotide sequence. In order to produce the protein of interest, either a second nucleotide sequence encoding the protein of interest is introduced into the platform plant using standard techniques involving either stable transformation, or transient transformation, and the second nucleotide sequence is expressed so that the protein of interest produced comprises glycans with modified N-glycosylation, or the plant expressing the first nucleotide sequence is crossed with a second plant that expresses the second nucleotide sequence and in this manner the protein of interest produced comprises glycans with modified N-glycosylation. The protein of interest may be extracted from the plant, and if desired, the protein of interest may be isolated and purified using standard methods.

Therefore, disclosed is a method (Method J) for producing a plant that may be used as a platform for the production of a protein of interest with modified N-glycosylation. This method comprises, providing a plant that expresses one or more than one first nucleotide sequence encoding GnT-III, GNT1-GnT-III, or both GnT-III and GNT1-GNT-III, and expressing the one or more nucleotide sequence. In order to produce the protein of interest, either a second nucleotide sequence encoding the protein of interest is introduced into the platform plant using standard techniques involving either stable transformation, or transient transformation, and the second nucleotide sequence is expressed so that the protein of interest produced comprises glycans with modified N-glycosylation, or the plant expressing the first nucleotide sequence is crossed with a second plant that expresses the second nucleotide sequence and in this manner the protein of interest produced comprises glycans with modified N-glycosylation. The protein of interest may be extracted from the plant, and if desired, the protein of interest may be isolated and purified using standard methods.

Therefore, disclosed is a method (Method K) for producing a plant that may be used as a platform for the production of a protein of interest with modified N-glycosylation. This method comprises, providing a plant that expresses one or more than one first nucleotide sequence encoding GalT, GNT1-GalT, GalT and GNT1-GalT, GnT-III, GNT1-GnT-III, GnT-III and GNT1-GNT-III, or a combination thereof, and expressing the one or more nucleotide sequence. In order to produce the protein of interest, either a second nucleotide sequence encoding the protein of interest is introduced into the platform plant using standard techniques involving either stable transformation, or transient transformation, and the second nucleotide sequence is expressed so that the protein of interest produced comprises glycans with modified N-glycosylation, or the plant expressing the first nucleotide sequence is crossed with a second plant that expresses the second nucleotide sequence and in this manner the protein of interest produced comprises glycans with modified N-glycosylation. The protein of interest may be extracted from the plant, and if desired, the protein of interest may be isolated and purified using standard methods.

The nucleotide sequences encoding GalT, GNT1-GalT, GnT-III, GNT1-GnT-III, the protein of interest, or a combination thereof may be codon optimized to increase the level of expression within the plant. By codon optimization it is meant the selection of appropriate DNA nucleotides for the synthesis of oligonucleotide building blocks, and their subsequent enzymatic assembly, of a structural gene or fragment thereof in order to approach codon usage within plants.

In order to optimize the expression of the foreign sequence within a plant, the nucleotide sequence, which may be a wild type or synthetic sequence may be used or altered as required so that the corresponding protein, for example GalT, GNT1-GalT, GnT-III, GNT1-GnT-III, the protein of interest, or a combination thereof, is produced at a level higher than would be produced when encoded by the un-modified nucleotide sequence. For example, which is not to be considered limiting, the sequence may be a synthetic sequence, optimized for codon usage within a plant, comprising at least about 80% identity with the wild type sequence, as determined using sequence comparison techniques for example but not limited to BLAST (available through GenBank; using default parameters). It is also contemplated that fragments or portions of the sequence encoding the protein of interest, or derivatives thereof, that exhibit useful biological properties, for example but not limited to antigenic properties, may be expressed within plant tissues.

In order to maximize expression levels and transgene protein production of GalT, GNT1-GalT, GnT-III, GNT1-GnT-III, and a protein of interest, the nucleic acid sequence may be examined and the coding region modified to optimize for expression of the gene in plants, using a procedure similar to that outlined by Sardana et al. (Plant Cell Reports 15:677-681; 1996). A table of codon usage from highly expressed genes of dicotyledonous plants is available from several sources including Murray et al. (Nuc Acids Res. 17:477-498; 1989). Furthermore, sequence optimization may also include the reduction of codon tandem repeats, the elimination of cryptic splice sites, the reduction of repeated sequence (including inverted repeats) and can be determined using, for example, Leto 1.0 (Entelechon, Germany).

Therefore, disclosed is a method (L) of synthesizing a protein of interest with modified N-glycosylation, as described in any one of the above methods (Method A-K), wherein one or more than one of the nucleotide sequence encoding GalT, GNT1-GalT, GnT-III, GNT1-GnT-III, the protein of interest, or a combination thereof, are optimized for expression in a plant.

Furthermore, the present invention pertains to a plant, a plant cell, or a seed, comprising a nucleotide sequence encoding GNT1-GalT operatively linked with a regulatory region that is active in the plant. The plant, plant cell, or seed may further comprise a second nucleotide sequence encoding one or more than one of a protein of interest, the second nucleotide sequence operatively linked to one or more than one second regulatory region active within the plant. The first nucleotide sequence, the second nucleotide sequence, or both the nucleotide sequence and the second nucleotide sequence, may be codon optimized for expression within the plant, plant cell or plant seed.

By "operatively linked" it is meant that the particular sequences interact either directly or indirectly to carry out an intended function, such as mediation or modulation of gene expression. The interaction of operatively linked sequences may, for example, be mediated by proteins that interact with the operatively linked sequences. A transcriptional regulatory region and a sequence of interest are operably linked when the sequences are functionally connected so as to permit transcription of the sequence of interest to be mediated or modulated by the transcriptional regulatory region.

By the term "portion of a plant", it is meant any part derived from a plant, including the entire plant, tissue obtained from the plant for example but not limited to the leaves, the leaves and stem, the roots, the aerial portion including the leaves, stem and optionally the floral portion of the plant, cells or protoplasts obtained from the plant.

By the term "plant matter", it is meant any material derived from a plant. Plant matter may comprise an entire plant, tissue, cells, or any fraction thereof. Further, plant matter may comprise intracellular plant components, extracellular plant components, liquid or solid extracts of plants, or a combination thereof. Further, plant matter may comprise plants, plant cells, tissue, a liquid extract, or a combination thereof, from plant leaves, stems, fruit, roots or a combination thereof. Plant matter may comprise a plant or portion thereof which has not be subjected to any processing steps. However, it is also contemplated that the plant material may be subjected to minimal processing steps as defined below, or more rigorous processing, including partial or substantial protein purification using techniques commonly known within the art including, but not limited to chromatography, electrophoresis and the like.

By the term "minimal processing" it is meant plant matter, for example, a plant or portion thereof comprising a protein of interest which is partially purified to yield a plant extract, homogenate, fraction of plant homogenate or the like. Partial purification may comprise, but is not limited to disrupting plant cellular structures thereby creating a composition comprising soluble plant components, and insoluble plant components which may be separated for example, but not limited to, by centrifugation, filtration or a combination thereof. In this regard, proteins secreted within the extracellular space of leaf or other tissues could be readily obtained using vacuum or centrifugal extraction, or tissues could be extracted under pressure by passage through rollers or grinding or the like to squeeze or liberate the protein free from within the extracellular space. Minimal processing could also involve preparation of crude extracts of soluble proteins, since these preparations would have negligible contamination from secondary plant products. Further, minimal processing may involve aqueous extraction of soluble protein from leaves, followed by precipitation with any suitable salt. Other methods may include large scale maceration and juice extraction in order to permit the direct use of the extract.

The plant matter, in the form of plant material or tissue may be orally delivered to a subject. The plant matter may be administered as part of a dietary supplement, along with other foods, or encapsulated. The plant matter or tissue may also be concentrated to improve or increase palatability, or provided along with other materials, ingredients, or pharmaceutical excipients, as required.

It is contemplated that a plant comprising the protein of interest may be administered to a subject, for example an animal or human, in a variety of ways depending upon the need and the situation. For example, the protein of interest obtained from the plant may be extracted prior to its use in either a crude, partially purified, or purified form. If the protein is to be purified, then it may be produced in either edible or non-edible plants. Furthermore, if the protein is orally administered, the plant tissue may be harvested and directly feed to the subject, or the harvested tissue may be dried prior to feeding, or an animal may be permitted to graze on the plant with no prior harvest taking place. It is also disclosed to use the harvested plant tissues as a food supplement within animal feed. If the plant tissue is being feed to an animal with little or not further processing it is preferred that the plant tissue being administered is edible.

As described in more detail in the Examples, GalT, GNT1-GalT, and the protein of interest were introduced into plants in a transient manner. Immunological analysis, using appropriate antibodies, demonstrated that a protein of MWᵣ 150 kDa was present in the transformed cells (Figures 2, 3A and 3B). Furthermore GalT or GNT1-GalT was detectable in extracts obtained from plants expressing either construct, and altered N glycosylation of a protein of interest was observed when GNT1-GalT was expressed in the plant (Figure 6). Therefore, recombinantly expressed GlaT, or GNT1-GalT is biologically active *in planta*.

GalT-FLAG or GNT1-GalT-FLAG (i.e. GalT or GNT1-GalT tagged at its C-terminus with a FLAG epitope to allow immunodetection of the recombinant protein in transformants) were introduced into plants. Western-blot analysis, using anti-FLAG antibody, may be used to demonstrate that the appropriate protein is present in the transformed cells.

An "analogue" or "derivative" includes any substitution, deletion, or addition to the nucleotide sequence encoding GalT (SEQ ID NO:14), the catalytic domain of GalT (nucleotides 368-1198 of SEQ ID NO:14; encoding bold sequence Figure 5b), or GNT1-GalT (nucleotides 248- 1077 of SEQ ID NO:17; encoding bold sequence Figure 5d), provided that the sequence encodes a protein that modifies, or when fused to a CST of GalT modifies, the glycosylation profile of a protein of interest when expressed in a plant, for example reducing the fucosylation, xylosylation, or both, of glycans of the protein of interest, or increasing the galactosylation of the protein of interest when compared to the glycoslylation profile of the protein of interest produced in a plant, in the absence of ectopically expressed GalT (SEQ ID NO:14) or GNT1-GalT (SEQ ID NO:17). For example the protein encoded by the sequence may add a terminal galactose during N-glycan maturation. Derivatives, and analogues of nucleic acid sequences typically exhibit greater than 80% identity with, a nucleic acid sequence, for example from about 80-100% sequence identify, or from about 80, 82, 84, 86, 88, 90, 92, 94, 96, 98, 100% identity. Sequence identity, may be determined by use of the BLAST algorithm (GenBank: ncbi.nlm.nih.gov/cgi- bin/BLAST/), using default parameters (Program: blastn; Database: nr; Expect 10; filter: low complexity; Alignment: pairwise; Word size: 11). Analogs, or derivatives thereof, also include those nucleotide sequences that hybridize under stringent hybridization conditions (see Maniatis et al., in Molecular Cloning (A Laboratory Manual), Cold Spring Harbor Laboratory, 1982, p. 387-389, or Ausubel, et al. (eds), 1989, Current Protocols in Molecular Biology, Vol. 1, Green Publishing Associates, Inc., and John Wiley & Sons, Inc., New York, at p. 2.10.3) to any one of the GalT (SEQ ID NO:14), GNAT1-GalT (SEQ ID NO:18) sequences described herein, provided that the sequence encodes a protein that modifies the glycosylation profile of a protein of interest, for example reducing the fucosylation, xylosylation, or both, of glycans of the protein of interest, or increasing the galactosylation of the protein of interest when compared to the glycoslylation profile of the protein of interest produced in the absence of GalT (SEQ ID NO:14) or GNT1-GalT (SEQ ID NO:17). For example the protein encoded by the sequence may add a terminal galactose during N-glycan maturation. An example of one such stringent hybridization conditions may be hybridization with a suitable probe, for example but not limited to, a [gama-³²P]dATP labelled probe for 16-20 hrs at 65C in 7% SDS, 1mM EDTA, 0.5M Na₂HPO₄, pH 7.2. Followed by washing at 65C in 5% SDS, 1mM EDTA 40mM Na₂HPO₄, pH 7.2 for 30 min followed by washing at 65C in 1% SDS, 1mM EDTA 40mM Na₂HPO₄, pH 7.2 for 30 min. Washing in this buffer may be repeated to reduce background.

Similarly, disclosed is a modification to the nucleotide sequence encoding GnT-III (SEQ ID NO:16), the catalytic domain of GnT-III, or GNT1-GnT-III (SEQ ID NO:20), provided that the sequence encodes a protein that modifies, or when fused to a CST of GnT-III modifies, the glycosylation profile of a protein of interest when expressed in a plant, for example bisecting GlnAc and reducing the fucosylation, xylosylation, or both, of glycans of the protein of interest, when compared to the glycoslylation profile of the protein of interest produced in a plant, in the absence of ectopically expressed GnT-III (SEQ ID NO:16) or GNT1-GnT-III (SEQ ID NO:26). Derivatives, and analogues of nucleic acid sequences typically exhibit greater than 80% identity with, a nucleic acid sequence, for example from about 80-100% sequence identify, or from about 80, 82, 84, 86, 88, 90, 92, 94, 96, 98, 100% identity. Sequence identity, may be determined by use of the BLAST algorithm (GenBank: ncbi.nlm.nih.gov/cgi- bin/BLAST/), using default parameters (Program: blastn; Database: nr; Expect 10; filter: low complexity; Alignment: pairwise; Word size: 11). Analogs, or derivatives thereof, also include those nucleotide sequences that hybridize under stringent hybridization conditions (see Maniatis et al., in Molecular Cloning (A Laboratory Manual), Cold Spring Harbor Laboratory, 1982, p. 387-389, or Ausubel, et al. (eds), 1989, Current Protocols in Molecular Biology, Vol. 1, Green Publishing Associates, Inc., and John Wiley & Sons, Inc., New York, at p. 2.10.3) to any one of the GnT-III (SEQ ID NO:16, GNAT1-GnT-III (SEQ ID NO:26) sequences described herein, provided that the sequence encodes a protein that modifies the glycosylation profile of a protein of interest when compared to the glycoslylation profile of the protein of interest produced in the absence of GnT-III (SEQ ID NO:16) or GNT1-GnT-III (SEQ ID NO:26). An example of one such stringent hybridization conditions may be hybridization with a suitable probe, for example but not limited to, a [gama-³²P]dATP labelled probe for 16-20 hrs at 65C in 7% SDS, 1mM EDTA, 0.5M Na₂HPO₄, pH 7.2. Followed by washing at 65C in 5% SDS, 1mM EDTA 40mM Na₂HPO₄, pH 7.2 for 30 min followed by washing at 65C in 1% SDS, 1mM EDTA 40mM Na₂HPO₄, pH 7.2 for 30 min. Washing in this buffer may be repeated to reduce background.

By "regulatory region" "regulatory element" or "promoter" it is meant a portion of nucleic acid typically, but not always, upstream of the protein coding region of a gene, which may be comprised of either DNA or RNA, or both DNA and RNA. When a regulatory region is active, and in operative association, or operatively linked, with a gene of interest, this may result in expression of the gene of interest. A regulatory element may be capable of mediating organ specificity, or controlling developmental or temporal gene activation. A "regulatory region" includes promoter elements, core promoter elements exhibiting a basal promoter activity, elements that are inducible in response to an external stimulus, elements that mediate promoter activity such as negative regulatory elements or transcriptional enhancers. "Regulatory region", as used herein, also includes elements that are active following transcription, for example, regulatory elements that modulate gene expression such as translational and transcriptional enhancers, translational and transcriptional repressors, upstream activating sequences, and mRNA instability determinants. Several of these latter elements may be located proximal to the coding region.

In the context of this disclosure, the term "regulatory element" or "regulatory region" typically refers to a sequence of DNA, usually, but not always, upstream (5') to the coding sequence of a structural gene, which controls the expression of the coding region by providing the recognition for RNA polymerase and/or other factors required for transcription to start at a particular site. However, it is to be understood that other nucleotide sequences, located within introns, or 3' of the sequence may also contribute to the regulation of expression of a coding region of interest. An example of a regulatory element that provides for the recognition for RNA polymerase or other transcriptional factors to ensure initiation at a particular site is a promoter element. Most, but not all, eukaryotic promoter elements contain a TATA box, a conserved nucleic acid sequence comprised of adenosine and thymidine nucleotide base pairs usually situated approximately 25 base pairs upstream of a transcriptional start site. A promoter element comprises a basal promoter element, responsible for the initiation of transcription, as well as other regulatory elements (as listed above) that modify gene expression.

There are several types of regulatory regions, including those that are developmentally regulated, inducible or constitutive. A regulatory region that is developmentally regulated, or controls the differential expression of a gene under its control, is activated within certain organs or tissues of an organ at specific times during the development of that organ or tissue. However, some regulatory regions that are developmentally regulated may preferentially be active within certain organs or tissues at specific developmental stages, they may also be active in a developmentally regulated manner, or at a basal level in other organs or tissues within the plant as well. Examples of tissue-specific regulatory regions, for example see-specific a regulatory region, include the napin promoter, and the cruciferin promoter (Rask et al., 1998, J. Plant Physiol. 152: 595-599; Bilodeau et al., 1994, Plant Cell 14: 125-130). An example of a leaf-specific promoter includes the plastocyanin promoter (US 7,125,978; SEQ ID NO:23; Figure 1b).

An inducible regulatory region is one that is capable of directly or indirectly activating transcription of one or more DNA sequences or genes in response to an inducer. In the absence of an inducer the DNA sequences or genes will not be transcribed. Typically the protein factor that binds specifically to an inducible regulatory region to activate transcription may be present in an inactive form, which is then directly or indirectly converted to the active form by the inducer. However, the protein factor may also be absent. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory region may be exposed to an inducer by externally applying the inducer to the cell or plant such as by spraying, watering, heating or similar methods. Inducible regulatory elements may be derived from either plant or non-plant genes (e.g. Gatz, C. and Lenk, I.R.P., 1998, Trends Plant Sci. 3, 352-358). Examples, of potential inducible promoters include, but not limited to, tetracycline-inducible promoter (Gatz, C.,1997, Ann. Rev. Plant Physiol. Plant Mol. Biol. 48, 89-108), steroid inducible promoter (Aoyama, T. and Chua, N.H.,1997, Plant J. 2, 397-404) and ethanol-inducible promoter (Salter, M.G., et al, 1998, Plant Journal 16, 127-132; Caddick, M.X., et al, 1998, Nature Biotech. 16, 177-180) cytokinin inducible IB6 and CKI1 genes (Brandstatter, I. and Kieber, J.J.,1998, Plant Cell 10, 1009-1019; Kakimoto, T., 1996, Science 274, 982-985) and the auxin inducible element, DR5 (Ulmasov, T., et al., 1997, Plant Cell 9, 1963-1971. A constitutive regulatory region directs the expression of a gene throughout the various parts of a plant and continuously throughout plant development. Examples of known constitutive regulatory elements include promoters associated with the CaMV 35S transcript. (Odell et al., 1985, Nature, 313: 810-812), the rice actin 1 (Zhang et al, 1991, Plant Cell, 3: 1155-1165), actin 2 (An et al., 1996, Plant J., 10: 107-121), or tms 2 (U.S. 5,428,147), and triosephosphate isomerase 1 (Xu et. al., 1994, Plant Physiol. 106: 459-467) genes, the maize ubiquitin 1 gene (Cornejo et al, 1993, Plant Mol. Biol. 29: 637-646), the *Arabidopsis* ubiquitin 1 and 6 genes (Holtorf et al, 1995, Plant Mol. Biol. 29: 637-646), and the tobacco translational initiation factor 4A gene (Mandel et al, 1995 Plant Mol. Biol. 29: 995-1004). The term "constitutive" as used herein does not necessarily indicate that a gene under control of the constitutive regulatory region is expressed at the same level in all cell types, but that the gene is expressed in a wide range of cell types even though variation in abundance is often observed.

The one or more than one nucleotide sequence of the present invention may be expressed in any suitable plant host that is transformed by the nucleotide sequence, or constructs, or vectors of the present invention. Examples of suitable hosts include, but are not limited to, agricultural crops including alfalfa, canola, *Brassica* spp., maize, *Nicotiana* spp., alfalfa, potato, ginseng, pea, oat, rice, soybean, wheat, barley, sunflower, and cotton.

The one or more chimeric genetic constructs of the present invention can further comprise a 3' untranslated region. A 3' untranslated region refers to that portion of a gene comprising a DNA segment that contains a polyadenylation signal and any other regulatory signals capable of effecting mRNA processing or gene expression. The polyadenylation signal is usually characterized by effecting the addition of polyadenylic acid tracks to the 3' end of the mRNA precursor. Polyadenylation signals are commonly recognized by the presence of homology to the canonical form 5' AATAAA-3' although variations are not uncommon. One or more of the chimeric genetic constructs of the present invention can also include further enhancers, either translation or transcription enhancers, as may be required. These enhancer regions are well known to persons skilled in the art, and can include the ATG initiation codon and adjacent sequences. The initiation codon must be in phase with the reading frame of the coding sequence to ensure translation of the entire sequence.

Non-limiting examples of suitable 3' regions are the 3' transcribed non-translated regions containing a polyadenylation signal of *Agrobacterium* tumor inducing (Ti) plasmid genes, such as the nopaline synthase (Nos gene) and plant genes such as the soybean storage protein genes and the small subunit of the ribulose-1, 5-bisphosphate carboxylase (ssRUBISCO) gene.

To aid in identification of transformed plant cells, the constructs of this invention may be further manipulated to include plant selectable markers. Useful selectable markers include enzymes that provide for resistance to chemicals such as an antibiotic for example, gentamycin, hygromycin, kanamycin, or herbicides such as phosphinothrycin, glyphosate, chlorosulfuron, and the like. Similarly, enzymes providing for production of a compound identifiable by colour change such as GUS (beta-glucuronidase), or luminescence, such as luciferase or GFP, may be used.

Also considered part of this invention are transgenic plants, plant cells or seeds containing the chimeric gene construct of the present invention. Methods of regenerating whole plants from plant cells are also known in the art. In general, transformed plant cells are cultured in an appropriate medium, which may contain selective agents such as antibiotics, where selectable markers are used to facilitate identification of transformed plant cells. Once callus forms, shoot formation can be encouraged by employing the appropriate plant hormones in accordance with known methods and the shoots transferred to rooting medium for regeneration of plants. The plants may then be used to establish repetitive generations, either from seeds or using vegetative propagation techniques. Transgenic plants can also be generated without using tissue cultures.

The regulatory elements described may also be combined with coding region of interest for expression within a range of host organisms that are amenable to transformation, or transient expression. Such organisms include, but are not limited to plants, both monocots and dicots, for example but not limited to corn, cereal plants, wheat, barley, oat, *Nicotiana* spp, *Brassica* spp, soybean, bean, pea, alfalfa, potato, tomato, ginseng, and *Arabidopsis.*

Methods for stable transformation, and regeneration of these organisms are established in the art and known to one of skill in the art. The method of obtaining transformed and regenerated plants is not critical to the present invention.

By "transformation" it is meant the interspecific transfer of genetic information (nucleotide sequence) that is manifested genotypically, phenotypically, or both. The interspecific transfer of genetic information from a chimeric construct to a host may be heritable and the transfer of genetic information considered stable, or the transfer may be transient and the transfer of genetic information is not inheritable.

Disclosed is a suitable vector comprising the chimeric construct suitable for use with either stable or transient expression systems. The genetic information may be also provided within one or more than one construct. For example, a nucleotide sequence encoding a protein of interest may be introduced in one construct, and a second nucleotide sequence encoding a protein that modifies glycosylation of the protein of interest may be introduced using a separate construct. These nucleotide sequences may then be co-expressed within a plant. However, a construct comprising a nucleotide sequence encoding both the protein of interest and the protein that modifies glycosylation profile of the protein of interest may also be used. In this case the nucleotide sequence would comprise a first sequence comprising a first nucleic acid sequence encoding the protein of interest operatively linked to a promoter or regulatory region, and a second sequence comprising a second nucleic acid sequence encoding the protein that modifies the glycosylation profile of the protein of interest, the second sequence operatively linked to a promoter or regulatory region.

By "co-expressed" it is meant that two or more than two nucleotide sequences are expressed at about the same time within the plant, and within the same tissue of the plant. However, the nucleotide sequences need not be expressed at exactly the same time. Rather, the two or more nucleotide sequences are expressed in a manner such that the encoded products have a chance to interact. For example, the protein that modifies glycosylation of the protein of interest may be expressed either before or during the period when the protein of interest is expressed so that modification of the glycosylation of the protein of interest takes place. The two or more than two nucleotide sequences can be co-expressed using a transient expression system, where the two or more sequences are introduced within the plant at about the same time under conditions that both sequences are expressed. Alternatively, a platform plant comprising one of the nucleotide sequences, for example the sequence encoding the protein that modifies the glycosylation profile of the protein of interest, may be transformed, either transiently or in a stable manner, with an additional sequence encoding the protein of interest. In this case, the sequence encoding the protein that modifies the glycosylation profile of the protein of interest may be expressed within a desired tissue, during a desired stage of development, or its expression may be induced using an inducible promoter, and the additional sequence encoding the protein of interest may be expressed under similar conditions and in the same tissue, to ensure that the nucleotide sequences are co-expressed.

The constructs can be introduced into plant cells using Ti plasmids, Ri plasmids, plant virus vectors, direct DNA transformation, micro-injection, electroporation, etc. For reviews of such techniques see for example Weissbach and Weissbach, Methods for Plant Molecular Biology, Academy Press, New York VIII, pp. 421-463 (1988); Geierson and Corey, Plant Molecular Biology, 2d Ed. (1988); and Miki and Iyer, Fundamentals of Gene Transfer in Plants. In Plant Metabolism, 2d Ed. DT. Dennis, DH Turpin, DD Lefebrve, DB Layzell (eds), Addison Wesly, Langmans Ltd. London, pp. 561-579 (1997). Other methods include direct DNA uptake, the use of liposomes, electroporation, for example using protoplasts, micro-injection, microprojectiles or whiskers, and vacuum infiltration. See, for example, Bilang, et al. (Gene 100: 247-250 (1991), Scheid et al. (Mol. Gen. Genet. 228: 104-112, 1991), Guerche et al. (Plant Science 52: 111-116, 1987), Neuhause et al. (Theor. Appl Genet. 75: 30-36, 1987), Klein et al., Nature 327: 70-73 (1987); Howell et al. (Science 208: 1265, 1980), Horsch et al. (Science 227: 1229-1231, 1985), DeBlock et al., Plant Physiology 91: 694-701, 1989), Methods for Plant Molecular Biology (Weissbach and Weissbach, eds., Academic Press Inc., 1988), Methods in Plant Molecular Biology (Schuler and Zielinski, eds., Academic Press Inc., 1989), Liu and Lomonossoff (J. Virol Meth, 105:343-348, 2002,), U.S. Pat. Nos. 4,945,050; 5,036,006; and 5,100,792, U.S. patent application Ser. Nos. 08/438,666, filed May 10, 1995, and 07/951,715, filed Sep. 25, 1992.

As described below, transient expression methods areused to express the constructs of the present invention (see Liu and Lomonossoff, 2002, Journal of Virological Methods, 105:343-348). Alternatively, a vacuum-based transient expression method, as described by Kapila et al. (1997) may be used. These methods may include, for example, but are not limited to, a method of Agro-inoculation or Agro-infiltration, however, other transient methods may also be used as noted above. With either Agro-inoculation or Agro-infiltration, a mixture of *Agrobacteria* comprising the desired nucleic acid enter the intercellular spaces of a tissue, for example the leaves, aerial portion of the plant (including stem, leaves and flower), other portion of the plant (stem, root, flower), or the whole plant. After crossing the epidermis the *Agrobacteria* infect and transfer t-DNA copies into the cells. The t-DNA is episomally transcribed and the mRNA translated, leading to the production of the protein of interest in infected cells, however, the passage of t-DNA inside the nucleus is transient.

By "gene of interest", "nucleotide sequence of interest", or "coding region of interest", it is meant any gene, nucleotide sequence, or coding region that is to be expressed within a host organism, for example a plant. These terms are used interchangeably. Such a nucleotide sequence of interest may include, but is not limited to, a gene or coding region whose product is a protein of interest. Examples of a protein of interest include, for example but not limited to, an industrial enzyme, a protein supplement, a nutraceutical, a value-added product, or a fragment thereof for feed, food, or both feed and food use, a pharmaceutically active protein, for example but not limited to growth factors, growth regulators, antibodies, antigens, and fragments thereof, or their derivatives useful for immunization or vaccination and the like. Additional proteins of interest may include, but are not limited to, interleukins, for example one or more than one of IL-1 to IL-24, IL-26 and IL-27, cytokines, Erythropoietin (EPO), insulin, G-CSF, GM-CSF, hPG-CSF, M-CSF or combinations thereof, interferons, for example, interferon-alpha, interferon-beta, interferon-gama, blood clotting factors, for example, Factor VIII, Factor IX, or tPA hGH, receptors, receptor agonists, antibodies, neuropolypeptides, insulin, vaccines, growth factors for example but not limited to epidermal growth factor, keratinocyte growth factor, transformation growth factor, growth regulators, antigens, autoantigens, fragments thereof, or combinations thereof.

If the nucleotide sequence of interest encodes a product that is directly or indirectly toxic to the plant, then by using the method of the present invention, such toxicity may be reduced throughout the plant by selectively expressing the gene of interest within a desired tissue or at a desired stage of plant development. In addition, the limited period of expression resulting from transient expression may reduce the effect when producing a toxic product in the plant.

The coding region of interest or the nucleotide sequence of interest may be expressed in any suitable plant host which is either transformed or comprises the nucleotide sequences, or nucleic acid molecules, or genetic constructs, or vectors of the present invention. Examples of suitable hosts include, but are not limited to, *Arabidopsis,* agricultural crops including for example canola, *Brassica* spp., maize, *Nicotiana* spp., alfalfa, potato, ginseng, pea, oat, rice, soybean, wheat, barley, sunflower, and cotton.

As described in more detail in the examples below, synthesis of a protein of interest, for example but not limited to an antibody, C5-1, with a modified N-glycosylation was produced in a plant co-expressing either GalT, or GNT1-GalT.

**Listing of sequences:**

| Sequence | SEQ ID NO: | Sequence | SEQ ID NO: |
|---|---|---|---|
| XmaI-pPlas.c | 1 | GNT1-GalT (nucleotide sequence) | 17 |
| SacI-ATG-pPlas.r | 2 | GNT1-GalT amino acid | 18 |
| SacI-PlasTer.c | 3 | GnT-III amino acid | 19 |
| EcoRI-PlasTer.r | 4 | GNT1-GnT-III amino acid | 20 |
| Plasto-443c | 5 | CTS domain of GNT1 (nucleotide) | 21 |
| Plas+LC-C51.r | 6 | CTS domain of GNT1 (amino acid) | 22 |
| LC-C51.c | 7 | Plastocyanin promoter and 5'UTR | 23 |
| LC-C51XhoSac.r | 8 | Plastocyanin 3' UTR and terminator | 24 |
| Plas+HC-C51.r | 9 | FgalTSpe | 25 |
| HC-C51.c | 10 | GNT1-GnT-III (nucleotide) | 26 |
| HC-C51XhoSac.r | 11 | FgalT | 27 |
| HC-C51KDEL(SacI).r | 12 | RgalTFlagStu | 28 |
| Tryptic glycopeptide | 13 | FGNT | 29 |
| GalT I (nucleotide) | 14 | RGBTSpe | 30 |
| GalT I amino acid | 15 | | |
| GnT-III (nucleotide) | 16 | | |

### Examples

### Example 1: Assembly of expression cassettes R610, R612 (Figure 1a), R621 and R622 (Figure 5a)

All manipulations were done using general molecular biology protocols from Sambrook and Russel (2001).

### R610, R612 (Figure 1a)

Oligonucleotide primers used are presented below:
**XmaI-pPlas.c:** SEQ ID NO:1 5'-AGTTCCCCGGGCTGGTATATTTATATGTTGTC-3' SEQ ID NO:1
**SacI-ATG-pPlas.r:** SEQ ID NO:2 5'-AATAGAGCTCCATTTTCTCTCAAGATGATTAATTAATTAATTAGTC-3' SEQ ID NO :2
**SacI-PlasTer.c:** SEQ ID NO:3 5'-AATAGAGCTCGTTAAAATGCTTCTTCGTCTCCTATTTATAATATGG-3' SEQ ID NO:3
**EcoRI-PlasTer.r:** SEQ ID NO:4 5'-TTACGAATTCTCCTTCCTAATTGGTGTACTATCATTTATCAAAGGGGA-3' SEQ ID NO:4
**Plasto**-**443c:** SEQ ID NO:5 5'-GTATTAGTAATTAGAATTTGGTGTC-3' SEQ ID NO:5
**Plas+LC-C51.r:** SEQ ID NO:6 5'-ATCTGAGGTGTGAAAACCATTTTCTCTCAAGATG-3' SEQ ID NO:6
**LC**-**C51**.**c:** SEQ ID NO:7 5'-ATGGTTTTCACACCTCAGATACTTGG-3' SEQ ID NO:7
**LC**-**C51XhoSac**.**r:** SEQ ID NO:8 5'-ATAT**GAGCTCCTCGAGC**TAACACTCATTCCTGTTGAAGC-3' SEQ ID NO:8
**Plas+HC-C51.r:** SEQ ID NO:9 5'-CAAGGTCCACACCCAAGCCATTTTCTCTCAAGATG-3' SEQ ID NO:9
**HC**-**C51**.**c:** SEQ ID NO:10 5'-ATGGCTTGGGTGTGGACCTTGC-3' SEQ ID NO:10
**HC**-**C51XhoSac**.**r:** SEQ ID NO:11 5'-ATAA**GAGCTCCTCGAG**TCATTTACCAGGAGAGTGGG-3' SEQ ID NO:11
**HC-C51*KDEL*(SacI).r:** SEQ ID NO:12 5'-ATAA**GAGCTC**TCA*AAGTTCATCCTT*TTTACCAGGAGAGTGGG-3' SEQ ID NO:12

The first cloning step consisted in assembling a receptor plasmid containing upstream and downstream regulatory elements of the alfalfa plastocyanin gene. The plastocyanin promoter (US Pat. 7,125,978) and 5'UTR sequences were amplified from alfalfa genomic DNA using oligonucleotide primers XmaI-pPlas.c (SEQ ID NO:1) and SacI-ATG-pPlas.r (SEQ ID NO:2). The resulting amplification product was digested with XmaI and SacI and ligated into pCAMBIA2300, previously digested with the same enzymes, to create pCAMBIA-PromoPlasto. Similarly, the 3'UTR sequences and terminator, of the plastocyanin gene (Figure 1c; nucleotides 1-399 of SEQ ID NO:24) was amplified from alfalfa genomic DNA using the following primers: SacI-PlasTer.c (SEQ ID NO:3) and EcoRI-PlasTer.r, (SEQ ID NO:4) and the product was digested with SacI and EcoRI before being inserted into the same sites of pCAMBIA-PromoPlasto to create pCAMBIAPlasto.

Plasmids R 610 and R 612 were prepared so as to contain a C5-1 light-and a C5-1 heavy-chain coding sequences under the plastocyanin promoter of alfalfa as tandem constructs; R 610 was designed to allow retention in the ER of the assembled IgG and comprised KDEL sequence fused to the end of the heavy chain of C5-1, and R 612 was designed to allow secretion.

The assembly of C5-1 expression cassettes was performed using a PCR-mediated ligation method described by Darveau et al. (1995). To assemble the light chain coding sequences downstream of the plastocyanin promoter, a first step consisted in amplifying the first 443 base pairs (bp) of the alfalfa plastocyanin promoter (nucleotides 556-999 of Figure 1b or SEQ ID NO:23) described by D'Aoust et al. (US Pat. 7,125,978) downstream of the initial ATG by PCR using pCAMBIAPlasto as template and the following primers:
Plasto-443c (SEQ ID NO:5) and Plas+LC-C51.r (SEQ ID NO:6; overlap is underlined, above).

In parallel, the light chain coding sequence was PCR-amplified from plasmid pGA643-kappa (Khoudi et al., 1999) with primers the following primers:
LC-C51.c (SEQ ID NO: 7) and LC-C51XhoSac.r. (SEQ ID NO:8; overlap is underlined).

The two amplification products obtained were mixed together and used as template in a third PCR reaction using primers Plasto-443c (SEQ ID NO:5) and LC-C51XhoSac.r (SEQ ID NO:8). The overlap between primers Plas+LC-C51.r (SEQ ID NO:6) and LC-C51.c (SEQ ID NO:7) used in the first reactions lead to the assembly of the amplification products during the third reaction. The assembled product resulting from the third PCR reaction was digested with DraIII and SacI and ligated in pCAMBIAPlasto digested with DraIII and SacI to generate plasmid R540.

The heavy chain coding sequence was fused to plastocyanin upstream regulatory element by amplifying the 443 bp upstream of the initial ATG of plastocyanin, nucleotides 556-999 of (Figure 1b; SEQ ID NO:23), by PCR using pCAMBIAPlasto as template with the following primers:
Plasto-443c (SEQ ID NO:5) and Plas+HC-C51.r (SEQ ID NO:9; overlap underlined above).

The product of these reactions were mixed and assembled in a third PCR reaction using primers Plasto-443c (SEQ ID NO:5) and HC-C51XhoSac.r (SEQ ID NO:11). The resulting fragment was digested with DraIII and SacI and ligated in pCAMBIAPlasto between the DraIII and SacI sites. The resulting plasmid was named R541.

A KDEL tag was added in C-terminal of the heavy chain coding sequence by PCR-amplification with primers Plasto-443c (SEQ ID NO:8) and HC-C51*KDEL* (SacI).r (SEQ ID NO:12) using plasmid R541 as a template. The resulting fragment was digested with DraIII and SacI cloned into the same sites of pCAMBIAPlasto, creating plasmid R550.

Assembly of light- and heavy chain expression cassettes on the same binary plasmid was performed as follows: R541 and R550 were digested with EcoRI, blunted, digested with HindIII and ligated into the HindIII and SmaI sites of R540 to create R610 (with KDEL) and R612 (without KDEL; see Figure 1).

*R621 and R622 (**Figure 5a*) - Oligonucleotide primers used are presented below:
**FgalT** SEQ ID NO:27 5'-GACTCTAGAGCGGGAAGATGAGGCTTCGGGAGCCGCTC-3' SEQ ID NO:27
**RgalTFlagStu** SEQ ID NO:28 
5'- AAGGCCTACG CTACTTGTCAT CGTCATCTTT GTAGTCGCAC
GGTGTCCCG AAGTCCAC -3' SEQ ID NO: 28
**FGNT** SEQ ID NO:29 5'-ATCGAAATCGCACGATGAGAGGGAACAAGTTTTGC-3' SEQ ID NO: 29
**RGNTSpe** SEQ ID NO:30 5'-CGGGATCCACTAGTCTGACGCTTCATTTGTTCTTC-3' SEQ ID NO: 30
**FgalTSpe** SEQ ID NO:25 5'-GGACTAGTGCACTGTCGCTGCCCGCCTGC-3' SEQ ID NO: 25

Plasmids for GalT and GNTIGalT expression were assembled from pBLTIl21 (Pagny et al., 2003). The human β(1,4)-galactosyltransferase (hGalT) gene (UDP galactose: β-N-acetylglucosaminide: β(1,4)-galactosyltransferase; EC 2.4.1.22) was isolated from pUC19-hGalT (Watzele et al., 1991) with EcoRI digestion. After klenow treatment, the 1.2-kb hGalT fragment was cloned into pBLTI221 at Sma I sites, resulting in plasmid pBLTI221hGalT. A flag tag was then fused to the C-terminal end of the coding region by PCR using primers FGalT (SEQ ID NO: 27) and RGalTFlagStu (SEQ ID NO: 28) for amplification. R622 was then produced by cloning this XbaI-StuI fragment into the binary vector pBLTI121. The first 77 a.a. from N-acetylglucosaminyltransferase I (GNTI) corresponding to the transmembrane domain were amplified by PCR using the *N. tabacum* cDNA encoding N-GNTI as template (Strasser et al, 1999) and FGNT (SEQ ID NO: 29) and RGNTSpe (SEQ ID NO: 30) as primers. The amplification product was first cloned into pGEM-T vector, and the resulting plasmid was digested with ApaI and BamHI, and ligated into pBLTI221, producing a plasmid named pBLTI221-GNTI. The catalytic domain of hGalT was obtained by PCR amplification on pBLTI221hGalT using primers FGalTSpe (SEQ ID NO: 25) and RgalTFlagStu (SEQ ID NO: 28), creating SpeI and StuI sites in 5' and 3' end, respectively. The SpeI/StuI hGalT fragment was then cloned into pBLTI221-GNTI using the same (SpeI and StuI) sites, creating pBLTI221-GNTIGalT. Finally, pBLTI221-GNTIGalT was digested with XbaI and StuI isolating the GNTIGalT coding sequence (Figure 5d; SEQ ID NO: 17), and R621 was produced by cloning this fragment into the binary vector pBLTI121.

All clones were sequenced to confirm the integrity of the constructs. The plasmids were used to transform *Agrobacteium tumefaciens* (AGL1; ATCC, Manassas, VA 20108, USA) by electroporation (Höfgen and Willmitzer, 1988) using a Gene Pulser II apparatus (Biorad, Hercules, CA, USA) as for E. coli transformation (W.S. Dower, Electroporation of bacteria, In "Genetic Engineering", Volume 12, Plenum Press, New York, 1990, J.K. Setlow eds.). The integrity of all A. *tumefaciens* strains were confirmed by restriction mapping.

An HcPro construct was prepared as described in Hamilton et al. (2002).

### Example 2: Preparation of plant biomass, inoculum, agroinfiltration, and harvesting

*Nicotiana benthamiana* plants were grown from seeds in flats filled with a commercial peat moss substrate. The plants were allowed to grow in the greenhouse under a 16/8 photoperiod and a temperature regime of 25°C day/20°C night. Three weeks after seeding, individual plantlets were picked out, transplanted in pots and left to grow in the greenhouse for three additional weeks under the same environmental conditions.

*Agrobacteria* strains R612, R610, .R621, R622 or 35SHcPro were grown in a YEB medium supplemented with 10 mM 2-[N-morpholino]ethanesulfonic acid (MES), 20 µM acetosyringone, 50 µg/ml kanamycin and 25 µg/ml of carbenicillin pH5.6 until they reached an OD₆₀₀ between 0.6 and 1.6. *Agrobacterium* suspensions were centrifuged before use and resuspended in infiltration medium (10 mM MgCl2 and 10 mM MES pH5.6).

Syringe-infiltration was performed as described by Liu and Lomonossoff (2002, Journal of Virological Methods, 105:343-348)).

For vacuum-infiltration, A. *tumefaciens* suspensions were centrifuged, resuspended in the infiltration medium and stored overnight at 4°C. On the day of infiltration, culture batches were diluted in 2.5 culture volumes and allowed to warm before use. Whole plants of *Nicotiana benthamiana* were placed upside down in the bacterial suspension in an air-tight stainless steel tank under a vacuum of 20-40 Torr for 2-min. Following syringe or vacuum infiltration, plants were returned to the greenhouse for a 4-5 day incubation period until harvest.

### Leaf sampling and total protein extraction

Following incubation, the aerial part of plants was harvested, frozen at -80°C, crushed into pieces and separated into 1.5 or 7.5 g sub-samples. Total soluble proteins were extracted by homogenizing (Polytron) each sub-sample of frozen-crushed plant material in 3 volumes of cold 50 mM Tris pH 7.4, 0.15 M NaCl, 0.1% Triton X-100, 1 mM phenylmethanesulfonyl fluoride and 10 µM chymostatin. After homogenization, the slurries were centrifuged at 20,000 g for 20 min at 4°C and these clarified crude extracts (supernatant) kept for analyses. The total protein content of clarified crude extracts was determined by the Bradford assay (Bio-Rad, Hercules, CA) using bovine serum albumin as the reference standard.

### Example 3: Protein analysis, Immunoblotting and ELISA

C5-1 is an anti-human murine IgG therefore detection and quantification can be performed through either its characteristic affinity to human IgGs (activity blots) or by its immunoreactivity to anti-mouse IgGs.

Proteins from total crude extracts or purified antibody were separated by SDS-PAGE and either stained with Coomassie Blue R-250 or G-250 or electrotransferred onto polyvinylene difluoride membranes (Roche Diagnostics Corporation, Indianapolis, IN) for immunodetection. Prior to immunoblotting, the membranes were blocked with 5% skim milk and 0.1% Tween-20 in Tris-buffered saline (TBS-T) for 16-18h at 4°C.

Immunoblotting was performed by incubation with the following antibodies: a peroxidase-conjugated goat anti-mouse IgG (H+L) antibody (Jackson ImmunoResearch, West Grove, PA, Cat# 115-035-146) (0.04 µg/ml in 2% skim milk in TBS-T), a peroxidase-conjugated human IgG antibody (Gamunex® Bayer Corp., Elkhart, IN) (0.2 µg/ml in 2% skim milk in TBS-T) or a polyclonal goat anti-mouse IgG antibody (heavy chain specific) (Sigma-Aldrich, St-Louis, MO) (0.25 µg/ml in 2% skim milk in TBS-T). A peroxidase-conjugated donkey anti-goat IgG antibody (Jackson ImmunoResearch) (0.04 µg/ml in 2% skim milk in TBS-T) was used as a secondary antibody for membranes treated with the heavy chain-specific antibody. Immunoreactive complexes were detected by chemiluminescence using luminol as the substrate (Roche Diagnostics Corporation). Horseradish peroxidase-enzyme conjugation of human IgG antibody was carried out by using the EZ-Link Plus^{®} Activated Peroxidase conjugation kit (Pierce, Rockford, IL).

### ELISA quantitative assay

Multiwell plates (Immulon 2HB, ThermoLab System, Franklin, MA) were coated with 2.5 µg/ml of goat anti-mouse antibody specific to IgG1 heavy chain (Sigma M8770) in 50 mM carbonate buffer (pH 9.0) at 4°C for 16-18h. Multiwell plates were then blocked through a 1h incubation in 1% casein in phosphate-buffered saline (PBS) (Pierce Biotechnology, Rockford, Il) at 37C. A standard curve was generated with dilutions of a purified mouse IgG1 control (Sigma M9269). When performing the immunoassays, all dilutions (control and samples) were performed in a plant extract obtained from plant tissue infiltrated and incubated with a mock inoculum so that any matrix effect be eliminated. Plates were incubated with protein samples and standard curve dilutions for 1h at 37 °C. After three washes with 0.1% Tween-20 in PBS (PBS-T), the plates were incubated with a peroxidase-conjugated goat anti-mouse IgG (H+L) antibody (0.04 µg/ml in blocking solution) (Jackson ImmunoResearch 115-035-146) for 1h at 37 °C. The washes with PBS-T were repeated and the plates were incubated with a 3,3', 5,5'-Tetramethylbenzidine (TMB) Sure Blue peroxidase substrate (KPL, Gaithersburg, MD). The reaction was stopped by adding 1N HCl and the absorbance was read at 450 nm. Each sample was assayed in triplicate and the concentrations were interpolated in the linear portion of the standard curve.

### Example 4: IgG purification

Purification of C5-1 from leaf material involved taking frozen leaves of *N. benthamiana* (100-150g), adding 20 mM sodium phosphate, 150 mM NaCl and 2 mM sodium meta-bisulfite at pH 5.8-6.0 and blending using a commercial blender for 2-3 min at room temperature. Insoluble fibres were removed by a coarse filtration on Miracloth ™ (Calbiochem, San Diego, CA) and 10 mM phenylmethanesulphonyl fluoride (PMSF) was added to the filtrate. The extract was adjusted to pH 4.8 ± 0.1 with 1 M HCl and clarified by centrifugation at 18 000 g for 15 min at 2-8°C. The clarified supernatant was adjusted to pH 8.0 ± 0.1 with 2 M TRIS, clarified again by centrifugation at 18 000 g for 15 min at 2-8°C, and filtered on sequential 0.8 and 0.2 µm membranes (Pall Corporation, Canada). The filtered material was concentrated by tangential flow filtration using a 100 kDa molecular weight cut-off ultrafiltration membrane of 0.2 ft² of effective area (GE Healthcare Biosciences, Canada) to reduce the volume of the clarified material by 5 to 10-fold. The concentrated sample was then applied to a 5mm x 5 cm column (1 mL column volume) of recombinant protein G-Sepharose Fast Flow (Sigma-Aldrich, St-Louis, MO, Cat. # P4691). The column was washed with 5 column volumes of 20 mM TRIS-HCl, 150mM NaCl pH 7.5. The antibody was eluted with 100mM Glycine pH 2.9-3.0, and immediately brought to neutral pH by collection into tubes containing calculated volumes of 1 M TRIS-HCl pH 7.5. The pooled fractions of eluted antibody were centrifuged at 21 000 g for 15 min at 2-8°C and stored at -80°C until analysis. After purification, the affinity column was cleaned and stored according to manufacturer's instructions. The same chromatographic media could be reused for several purifications without significant modification of purification performances (up to 10 cycles tested).

### Example 5: N-Glycosylation analysis

Samples comprising C5-1 (50 µg) were run on 15% SDS/PAGE. Heavy and light chains were revealed with Coomassie blue and the protein band corresponding to the heavy chain was excised and cut into small fragments. Fragments were washed 3 times with 600 µL of a solution of 0.1M NH4HCO3 / CH3CN (1/1) for 15 minutes each time and dried.

Reduction of disulfide bridges occurred by incubation of the gel fragments in 600 µL of a solution of 0.1M DTT in 0.1M NH4HCO3, at 56°C for 45 minutes. Alkylation was carried out by adding 600 µL of a solution of iodoacetamide 55 mM in 0.1M NH4HCO3, at room temperature for 30 minutes. Supernatants were discarded and polyacrylamide fragments were washed once again in NH4HCO3 0.1M / CH3CN (1/1).

Proteins were then digested with 7.5 µg of trypsin (Promega) in 600 µL of 0.05M NH4HCO3, at 37°C for 16 h. Two hundred µL of CH3CN were added and the supernatant was collected. Gel fragments were then washed with with 200 µL of 0.1M NH4HCO3, then with 200 µL CH3CN again and finally with 200 µL formic acid 5%. All supernatants were pooled and lyophilised.

Peptide separation by HPLC was carried out on a C18 reverse-phase column (4.5x250 mm) with a linear gradient of CH3CN in TFA 0.1%. Fractions were collected and lyophilised and analysed by MALDI-TOF-MS on a Voyager DE-Pro MALDI-TOF instrument (Applied Biosystems, USA) equipped with a 337-nm nitrogen laser. Mass spectra were performed in the reflector delayed extraction mode using alpha-cyano-4-hydroxycinnamic acid (Sigma-Aldrich) as matrix.

### Example 6: Quantification of transient IgG expression in agroinfiltrated Nicotiana berthamiana leaves.

To test the whether the strong plastocyanin-based expression cassettes could drive high accumulation of a fully assembled IgG, the coding sequences of the light and heavy chain of C5-1, a murine anti-human IgG (Khoudi et el 1997) were assembled in tandem constructs downstream of the plastocyanin promoter and 5' untranslated sequences, and flanked with the plastocyanin 3' untranslated and transcription termination sequences on the same T-DNA segment of a pCambia binary plasmid as presented in figure 1.

In both the R612 and R610 expression cassettes (see Example 1), the light and heavy chain coding sequences contained the native signal peptide from C5-1 (Khoudi et al. 1999), but in R610 the coding sequence of a KDEL peptide was added at the C-terminal of the heavy chain to restrain movement of the assembled IgG to the Golgi apparatus.

Following the cloning steps and the transfer of plasmids in *Agrobacterium tumefaciens* (AGL1), every leaf of three *Nicotiana benthamiana* plants were syringe-infiltrated with *Agrobacterium* strains transformed with plasmids R612 or R610, and incubated in greenhouse conditions for 6 days before analysis as described in Example 2. Following the incubation period, the leaves of each plant (approximately 20g of biomass) were frozen, ground, and the frozen powder was mixed to produce an homogenous sample from which 2 sub-samples of 1.5 grams were taken for extraction (from each plant; see Example 3). The content in C5-1 was quantified in total protein extracts from each sample by an enzyme-linked immunosorbent assay (ELISA) using a polyclonal goat anti-mouse IgG1 heavy chain for capture and a peroxidase-conjugated goat anti-mouse IgG (H+L) for detection (see Example 3).

As shown in figure 2, agroinfiltration of R612 led to the accumulation of 106 mg of antibody per kg of fresh weight, while the ER-retained form of the antibody (R610) reached 211 mg/kg FW in the same conditions.

Because post-transcriptional gene silencing (PTGS) has been shown to limit the expression of transgenes in agroinfiltrated *Nicotiana benthamiana* plants and that co-expression of a suppressor of silencing from the potato virus Y (HcPro) counteracted the specific degradation of transgene mRNAs (Brigneti et al., 1998), co-infiltration of an HcPro construct (Hamilton et al., 2002) was tested for its efficiency at increasing expression of C5-1. The co-expression of R612 and R610 with HcPro increased antibody accumulation levels by 5.3-fold and 3.6-fold, respectively, compared to these observed in the absence of HcPro. In the presence of HcPro, plastocyanin-controlled C5-1 expression reached average values of 558 mg/kg FW withR612, and 757 mg/kg FW with R610 (Figure 2). Maximum C5-1 expression levels exceeded 1.5 g/kg FW (25% of total soluble proteins) in some extracts form both R612- and R610- infiltrated leaves.

In order to assess the scalability of an agroinfiltration expression system, the accumulation of C5-1 was quantified following a vacuum infiltration procedure adapted from Kapila et al. (1997). In this series of experiments, the aerial part of whole plants were vacuum-infiltrated with R612 + HcPro or R610 + HcPro and returned to the greenhouse for 6 days before harvest. In an effort to provide data which are representative of a large-scale production system, batches of approximately 250 g lots of leaves/petioles from several plants were frozen, ground into an homogeneous sample, and 3 sub-samples of 7.5 grams of material per batch were collected for analysis. As shown by ELISA quantification, average C5-1 accumulation levels reached 238 and 328 mg /kg FW for R612 and R610 infiltrations respectively (Figure 2).

### Example 7: Characterization of the antibody produced

Protein blot analyses (see Example 3) were used to reveal the level of assembly and fragmentation of the C5-1 IgG in plants producing the secreted (R612) and ER-retained (R610) forms of the protein, following both syringe- and vacuum-infiltration experiments. A Western blot probed with a H+L peroxidase-conjugated goat anti-mouse IgG was first used to highlight the presence of a maximum of antibody fragments independently of their origin on the C5-1 molecule. As shown in figure 3A, all protein extracts contained fragments of similar molecular sizes and in similar relative abundance, irrespective of the subcellular targeting strategy or infiltration method used. In each case, a major band (≥85%) corresponding to the complete antibody at about 150 kDa, was revealed, with two minor bands at about135 kDa and about 100 kDa, showing that the antibody accumulated mainly in its fully assembled form (H₂L₂). Interestingly, fragments of similar electrophoretic mobility were also present in the control IgG1 purified from a murine tumor cell line (MOPC-21; Sigma #M9269), suggesting that the fragmentations produced in plants and mammalian cell lines were similar and probably resulted from common proteolytic activities. Similar results were also obtained with an anti-mouse heavy chain specific antibody for the detection.

To test the identity of the antibody fragments present in the extracts, an activity blot was used, in which the blotted proteins are probed with a peroxidase-conjugated human IgG1, the antigen of C5-1. The identity of a fully-assembled antibody of about 150 kDa band can be seen in Figure 3B. Furthermore, the fragmentation pattern observed in the Western blots, with the exception of a 100 kDa band (see Figure 3A) are visible on the activity blot (Figure 3B). Without wishing to be bound by theory, this result suggests that the 100kDA fragment does not contain the Fab regions of the C5-1 antibody, and may consist, at least in part, of dimers of heavy chains, an intermediate of antibody assembly.

### Example 8: Antibody purification and characterisation of the purified product

The antibody was purified from the biomass using a single Protein G affinity chromatographic step and the product obtained was analyzed by SDS-PAGE (see Example 4). The Coomassie stained gel presented in figure 4a shows a major band at 150 kDa in the eluate fraction from the Protein G. This band represents more than 85% of the purified product in both the secreted and ER-retained forms, and the contents in contaminants are identical for both forms (figure 4A, lanes 4 and 5). A Western blot analysis, probed with a polyclonal anti-mouse IgG, has shown the murine IgG origin of the major contaminant in the purified C5-1 fractions (data not shown). Under reducing conditions, two major products were detected at about 26 kDa and about 55 kDa which corresponds to the molecular weight of light and heavy chains, respectively (Figure 4B, lane 2). The heavy chain of the ER-retained antibody showed a higher electrophoretic mobility than the heavy chain of the apoplastic antibody (Figure 4B, lane 3) which is interpreted as the combined results of additional KDEL amino acids being present at the C-terminus and of differences in N-glycosylation due to the retention in the ER. Figure 4C shows that the purified antibodies (150 kDa) bound to human IgG1, as did contaminating fragments of 75, 90, 100, and 120 kDa , highlighting the presence of at least one Fab segment in these fragments. The presence of Fab in the 100 kDa fragment contrasted with the result obtained from crude extract analysis, where the 100 kDa band did not bind to human IgG. It is hypothesized that either the amount of Fab-containing fragments migrating at 100 kDa in the crude extract was too low for detection with this activity blot or that the fragment migrating at 100kDa consisted of two different molecules, one being heavy chain dimers (without Fab) and the other containing antigen-binding regions.

The reproducibility of this system for antibody production was assessed with a side-by-side comparison of purified products from 2 different infiltration batches and 3 distinct purification lots from each batch. The Coomassie-stained SDS-PAGE analysis of the purification lots showed the presence of identical bands in all lots, and in highly similar relative abundance (figure 4D).

### Example 9: Modification of antibody N-glycosylation by co-expression of a human glactosyltransferase

To investigate whether transient co-expression could be used to control glycosylation of nascent proteins during transient expression, plastocyanin expression cassettes comprising the native human β1,4galactosyltransferase (GalT) were prepared. R622 comprised GalT (Figure 5B), and R621 comprised GalT catalytic domain fused to the CTS domain of N-acetylglucosaminyl transferase (GNTI; GNT1GalT (Figure 5A). The CTS domain of N-acetylglucosaminyl transferase (GNTI) was selected as membrane anchorage for human GalT catalytic domain as GNT1 acts at an early stage of complex N-glycan synthesis in the ER and the cis-Golgi apparatus (Saint-Jore-Dupas et al., 2006). Without wishing to be limited by theory, sequestering GalT activity at an early stage of protein maturation may result in addition of β1,4galatose on maturating glycans and efficient inhibition of fucosylation and xylosylation of the core. These constructs were co-infiltrated in plants with C5-1.

*Nicotiana benthamiana* plant were infiltrated (see Example 2) with R612 (secreted form of C5-1), R612+R621(GNT1GalT) or R612+R622 (GalT) in the presence of HcPro. Figure 6 shows an immunological analysis of C5-1 purified from these biomass samples.

Galactosylation of the antibody was estimated by affinodetection with the *Erythrina cristagali* agglutinin (ECA) which specifically binds β1,4galactose. As expected, no galactose was detected when C5-1 was expressed alone (R612; Figure 6). Galactosylation was observed in C5-1 purified from co-infiltrations with R512+R622 (GalT) but not from co-infiltrations with R612+R621 (GNT1GalT, Figure 6). Western blot performed using anti-α1,3fucose antibodies revealed fucosylation of the N-glycan on the control C5-1 expressed without galactosyltransferase. Fucosylation of the N-glycan was not detected on the antibody co-expressed with GNTIGalT, irrespectively of the agroinfiltration method, whereas co-expression with the native GalT did not lead to detectable reduction in fucosylation of the antibody (Figure 6). Similar results were obtained with anti-β1,2xylose specific antibodies which showed the complete absence of xylose-specific immunosignals on C5-1 co-expressed with GNTIGalT and their presence when C5-1 was co-expressed with GalT (Figure 6).

Coomassie stained gels for direct visual estimates of fully-assembled IgG, and Western and activity blots were performed on the same extracts. Based on this data, the antibody expression system as described reaches yields of 1.5 g/kg fresh weight with over 85% of the product consisting of full-size tetrameric IgG of about 150 kDa in crude extracts.

The addition of a KDEL peptide at the C-terminal of the heavy chain has been used previously to increase antibody accumulation (2-10X) by mediating the retrieval of the antibody from the Golgi back to the ER (Schillberg et al., 2003). With the expression system described herein, the addition of a KDEL peptide to the heavy chain of C5-1 doubled the yield of C5-1 when the HcPro suppressor of silencing was not used. The difference between the yield of C5-1 in the presence or absence of KDEL was significantly reduced when HcPro was used to reduce silencing. ER-retention did not influence product quality since the fragments observed in the crude extracts from plants producing the ER-retained and secreted forms of the antibody were identical in size and relative abundance.

C5-1 was separated on preparative HPLC and the N-glycan profile of the tryptic glycopeptide EEQFNSTFR (SEQ ID NO:13) of C5-1 was analyzed by MALDI-TOF mass spectrometry. As shown in figure 7A, when C5-1 was expressed alone, its N-glycan population were mainly represented by complex forms, including ions consisting of fucosylated and xylosylated oligosaccharides as observed for stable expression (Bardor et al., 2003). The presence of non-mature ER-specific glycans, such as Man-8 and Man-9, can be related to a fraction of proteins "*en*-*route*" as previously reported for plant-derived antibody produced by transient expression (Sriraman, et al., 2004).

Co-expression of the antibody C5-1 with native GalT resulted in a significant modification of the N-glycan structure although ions corresponding to high-mannose-type N-glycans remained abundant. The main complex fucosylated and xylosylated N-glycan (J) disappeared and new partially galactosylated oligosaccharides were detected, some of them harbouring both plant-specific maturations and galactose extension such as GalGlcNAcMan₃(Xyl)GlcNAc₂ (Figure 7B). This demonstrated that co-expression of C5-1 with human β1,4galactosyltransferase resulted in an efficient glyco-engineering of the plant-derived antibody.

The co-expression of C5-1 with GNTIGalT yielded a purified C5-1 preparation in which the N-glycan population was significantly different than that of GalT/C5-1. As shown in figure 7C, galactosylated and non-galactosylated hybrids (GalGlcNAcMan₅GlcNAc₂ (K), and GalGlcNAcMan₅GlcNAc₂ (G), were present together with immature oligomannose N-glycans. Without wishing to be bound by theory, as hypothesized by Bakker et al. (2006), GlcNAcMan₅GlcNAc₂ (G) and Man₅GlcNAc₂ (B) may be fragments derived from degradation of the hybrid GalGlcNAcMan₅GlcNAc₂ by endogenous glycosidases rather than intermediates of mature N-glycan formation. The effect of the GNT1 membrane anchorage was striking; C5-1 purified from plants in which the synthetic enzyme GNT1GalT was co-expressed transiently with C5-1 contained no traces (≤99%) of glycans harboring the plant-specific α1,3fucose or β1,2xylose, demonstrating that complete modification of fucosylation and xylosylation was achieved during transient co-expression.

The present invention has been described with regard to one or more embodiments. However, it will be apparent to persons skilled in the art that a number of variations and modifications can be made without departing from the scope of the invention as defined in the claims.

### References:

Bakker, H. *et al.* An antibody produced in tobacco expressing a hybrid beta-1,4-galactosyltransferase is essentially devoid of plant carbohydrate epitopes. Proc. Natl. Acad. Sci. U.S.A. 103, 7577-7582 (2006).
Boisson, M, et al. Arabidopsis glucosidase I mutants reveal a critical role of N-glycan trimming in seed development. EMBO J. 20, 1010-1019 (2001).
Brigneti, G. et al. Viral pathogenicity determinants are suppressors of transgene silencing in Nicotiana benthamiana. EMBO J. 17, 6739-6746 (1998).
Chua, Y.L., Watson, L.A., & Gray, J.C. The transcriptional enhancer of the pea plastocyanin gene associates with the nuclear matrix and regulates gene expression through histone acetylation. Plant Cell 15, 1468-1479 (2003).
Cox, K.M., et al. Glycan optimization of a human monoclonal antibody in the aquatic plant Lemna minor. Nat. Biotechnol. 24, 1591-1597 (2006).
D'Aoust, M. A. et al. Efficient and reliable production of pharmaceuticals in alfalfa. Molecular Farming, pp 1-12. Rainer Fischer and Stefan Schillberg (eds.), Wiley-VCH, Weinheim, Germany (2004).
Darveau, A., Pelletier, A. & Perreault, J. PCR-mediated synthesis of chimeric molecules. Methods Neurosc. 26, 77-85 (1995).
Elmayan, T. & Vaucheret, H. Expression of single copies of a strongly expressed 35S transgene can be silenced post-transcriptionally. Plant J. 9, 787-797 (1996).
Fischer, R. et al. Towards molecular farming in the future: transient protein expression in plants. Biotechnol. Appl. Biochem. 30, 113-116 (1999).
Fischer, R., Drossard, J., Commandeur, U., Schillberg, S. & Emans, N. Towards molecular farming in the future: moving from diagnostic protein and antibody production in microbes to plants. Biotechnol. Appl. Biochem. 30, 101-108 (1999).
Giritch, A. et al. Rapid high-yield expression of full-size IgG antibodies in plants coinfected with noncompeting viral vectors. Proc. Natl. Acad. Sci. U.S.A. 103, 14701-14706 (2006).
Gomord, V., Chamberlain, P., Jefferis, R. & Faye, L. Biopharmaceutical production in plants: problems, solutions and opportunities. Trends Biotechnol. 23, 559-565 (2005).
Hamilton, A., Voinnet, O., Chappell, L. & Baulcombe, D. Two classes of short interfering RNA in RNA silencing. EMBO J. 21, 4671-4679 (2002).
Hiatt, A., Cafferkey, R. & Bowdish, K. Production of antibodies in transgenic plants. Nature 342, 76-78 (1989).
Hiatt, A. & Pauly, M. Monoclonal antibodies from plants: a new speed record. Proc. Natl. Acad. Sci. U.S.A. 103, 14645-14646 (2006).
Hibino, Y., Ohzeki, H., Sugano, N. & Hiraga, K. Transcription modulation by a rat nuclear scaffold protein, P130, and a rat highly repetitive DNA component or various types of animal and plant matrix or scaffold attachment regions. Biochem. Biophys. Res. Commun. 279, 282-287 (2000).
Höfgen, R. & Willmitzer, L. Storage of competent cells for Agrobacterium transformation. Nucleic Acid Res. 16, 9877 (1988).
Hull, A. K. et al. Human-derived, plant-produced monoclonal antibody for the treatment of anthrax. Vaccine 23, 2082-2086 (2005).
Kapila, J., De Rycke, R., Van Montagu, M. & Angenon, G. An Agrobacterium-mediated transient gene expression system for intact leaves. Plant Sci. 122, 101-108 (1997).
Kathuria, S.R. et al. Functionnal recombinant antibodies against human chorionic gonadotropin expressed in plants. Curr. Sci. 82, 1452-1456 (2002).
Ko, K. et al. Function and glycosylation of plant-derived antiviral monoclonal antibody. Proc. Natl. Acad. Sci. U.S.A. 100, 8013-8018 (2003).
Ko, K. & Koprowski, H. Plant biopharming of monoclonal antibodies. Virus Res. 111, 93-100 (2005).
Koprivova, A. et al. Targeted knockouts of physcomitrella lacking plant-specific immunogenic N-glycans. Plant Biotechnol. J. 2, 517-523 (2004).
Khoudi, H. et al. Production of a diagnostic monoclonal antibody in perennial alfalfa plants. Biotechnol. Bioeng. 64, 135-143 (1999).
Liu, L & Lomonossoff, G.P. Agroinfection as a rapid method for propagating Cowpea mosaic virus-based constructs. J. Virol. Methods 105, 343-348 (2002).
Ma, J. K-C., Drake, P.M.W., Chargelegue, D., Obregon, P. & Prada, A. Antibody processing and engineering in plants, and new strategies for vaccine production. Vaccine 23, 1814-1818 (2005).
Misaki, R., Fujiyama, K. & Seki, T. Expression of human CMP-N-acetylneuraminic acid synthetase and CMP-sialic acid transporter in tobacco suspension-cultured cell. Biochem. Biophys. Res Com. 339, 1184-1189 (2004).
Paccalet et al. Engineering of a sialic acid synthesis pathway in transgenic plants by expression of bacterial Neu5Ac-synthesizing enzymes. Plant Biotech. J. 5, (2007).
Pagny, S. et al. Protein recycling from the Golgi to the endoplasmic reticulum is very active in plants but has a minor contribution to calreticulin retention. Plant Cell 12, 739-752 (2000).
Pagny, S. et al. Structural requirements for Arabidopsis β1,2-xylosyltransferase activity and targeting to the Golgi. Plant J. 33, 189-203 (2003).
Peterson, E., Owens, S.M. & Henry, R. L. Monoclonal antibody form and function: manufacturing the right antibodies for treating drug abuse. AAPS J. 8, E383-E390 (2006).
Petrucelli, S. et al. A KDEL-tagged monoclonal antibody is efficiently retained in the endoplasmic reticulum in leaves, but is both partially secreted and sorted to protein storage vacuoles in seeds. Plant Biotechnol. J. 4, 511-527 (2006).
Pwee, K-H. & Gray, J.C. The pea plastocyanin promoter directs cell-specific but not full light-regulated expression in transgenic tobacco plants. Plant J. 3, 437-449 (1993).
Rodriguez, M. et al. Transient expression in tobacco leaves of an aglycosylated recombinant antibody against the epidermal growth factor receptor. Biotechnol. Bioeng. 89, 188-194 (2005).
Rouwendal, G.J.A. et al. Efficient introduction of a bisecting GlnAc residue in tobacco N-glycans by expression of the gene encoding human N-acetylglucosaminyltrasnferase III. Glycobiology 17, 334-344 (2007)
Saint-Jore-Dupas, C. et al. Plant N-glycan processing enzymes employ different targeting mechanisms for their spatial arrangement along the secretory pathway. Plant Cell 18, 3182-3200 (2006).
Sambrook J., and Russell, D.W., Molecular Cloning: A Labaratory Manual. Cold Spring Harbor Laboratory Press, Cold Spring Harbor, New York. (2001).
Sandhu, J.S., Webster, C.I., & Gray, J.C. A/T-rich sequences act as quantitative enhancers of gene expression in transgenic tobacco and potato plants. Plant Mol. Biol. 37, 885-896 (1998).
Schillberg, S., Fischer, R. & Emans, N. Molecular farming of recombinant antibodies in plants. Cell. Mol. Life Sci. 60, 433-445 (2003).
Sharp, J. M. & Doran, P. M. Characterization of monoclonal antibody fragments produced by plant cells. Biotechnol. Bioeng. 73, 338-346 (2001).
Sriraman, R. et al. Recombinant anti-hCG antibodies retained in the endoplasmic reticulum of transformed plants lack core-xylose and core-α(1-3)-fucose residues. Plant Biotechnol. J. 2, 279-287 (2004).
Strasser, R., Altmann, F., Mach, L., G16ssl, J. & Steinkellner, H. Generation of Arabidopsis thaliana plants with complex N-glycans lacking β1,2 linked xylose and core α1,3-linked fucose. FEBS Lett. 561, 132-136 (2004).
Szittya, G. et al. Low temperature inhibits RNA silencing-mediated defence by the control of siRNA generation. EMBO J. 22, 633-640 (2003).
Verch, T, Yusibov, V. & Koprowski, H. Expression and assembly of a full-length monoclonal antibody in plants using a plant virus vector. J. Immunol. Methods 220, 69-75 (1998).
Watzele, G.,Bachofner, R. & Berger, E.G.. Immunocytochemical localization of the Golgi apparatus using protein-specific antibodies to galactosyltransferase. Eur. J. Cell. Biol. 56, 451-458 (1991).
Yusibov, V., Rabindran, S., Commandeur, U. Twyman, R.M. & Fischer, R. The potential of plant virus vectors for vaccine production. Drugs R.D. 7, 203-217 (2006).

## Claims

1. A method for synthesizing a protein of interest with reduced fucosylation and xylosylation comprising, introducing within a plant, a portion of a plant, or a plant cell, a nucleotide sequence comprising from 80% to 100% identity with a nucleotide sequence defined by SEQ ID NO:17 and encoding a hybrid protein comprising a cytoplasmic tail, transmembrane domain, stem region (CTS domain) of N-acetylglucosaminyl transferase (GNT1) fused to a catalytic domain of beta-1,4galactosyltransferase (GalT), the first nucleotide sequence operatively linked with a first regulatory region that is active in the plant, and a second nucleotide sequence for encoding the protein of interest, the second nucleotide sequence operatively linked with a second regulatory region that is active in the plant, and transiently co-expressing the first and second nucleotide sequences to synthesize the protein of interest comprising less than 10% fucosylation and xylosylation when compared to the same protein of interest produced in a wild-type plant.

2. The method of claim 1 wherein the first regulatory region is a plastocyanin promoter, or a 35S promoter, and the second regulatory region is a plastocyanin promoter, or a 35S promoter.

3. The method of claim 1, wherein a third nucleotide sequence is expressed within the plant, the third nucleotide sequence encoding a suppressor of silencing, the third nucleotide sequence operatively linked with a third regulatory region that is active in the plant.

4. The method of claim 3, where the protein of interest is synthesized at an amount of up to 1.5g per kilogram of plant fresh weight.

5. The method of claim 3, wherein the third regulatory region is a plastocyanin promoter, or a 35S promoter.

6. The method of claim 2, wherein a third nucleotide sequence is expressed within the plant, the third nucleotide sequence encoding a suppressor of silencing, the third nucleotide sequence operatively linked with a third regulatory region that is active in the plant.

7. The method of claim 6, wherein the third regulatory region is a plastocyanin promoter, or a 35S promoter.

8. The method of claim 1, wherein the protein of interest is an antibody, an antigen or a vaccine.

9. The method of claim 8, wherein the second nucleotide sequence encoding the protein of interest comprises a nucleotide sequence 2A, operatively linked with a regulatory region 2A that is active in the plant, and a nucleotide sequence 2B, operatively linked with a regulatory region 2B that is active in the plant, and the product encoded by each of 2A and 2B combine to produce an antibody.

10. The method of claim 9, wherein the regulatory region 2A is a plastocyanin promoter, and the regulatory region 2B is a plastocyanin promoter.

11. A nucleic acid comprising nucleotides 1 to 1062 of SEQ ID NO:17 (GNT1-GalT), wherein the nucleotide sequence encodes a protein that modifies glycosylation of a protein of interest.

12. A hybrid protein GNT1-GalT, comprising a CTS domain of N-acetylglucosaminyl transferase fused to a catalytic domain of beta-1,4galactosyltransferase, the hybrid protein comprising amino acids 1 to 354 of sequence SEQ ID NO:18.

13. A plant, plant cell, or a seed comprising the nucleotide sequence of claim 11.

14. A plant, plant cell, or a seed comprising the hybrid protein of claim 12.

15. The method of claim 3, wherein the protein of interest is an antibody and synthesized at an amount of up to 1.5g per kilogram of leaf fresh weight.

16. The method of claim 1, wherein the synthesized protein of interest lacks fucosylation, xylosylation or both.

## Patentansprüche

1. Verfahren zum Synthetisieren eines Proteins von Interesse mit reduzierter Fukosylierung und Xylosylierung, umfassend Einführen in eine Pflanze, einen Teil einer Pflanze oder eine Pflanzenzelle einer Nukleotidsequenz, die von 80 % bis 100 % Identität mit einer durch SEQ ID NO:17 definierten Nukleotidsequenz umfasst und für ein Hybridprotein codiert, das einen Zytoplasmaschwanz, eine Transmembrandomäne, eine Stammregion (CTS-Domäne) von N-Acetylglucosaminyltransferase (GNT1) fusioniert an eine katalytische Domäne von beta-1,4-Galactosyltransferase (GalT) umfasst, wobei die erste Nukleotidsequenz in Betriebsbeziehung mit einer ersten Regulationsregion verknüpft ist, die in der Pflanze aktiv ist, und einer zweiten Nukleotidsequenz zum Codieren für das Protein von Interesse, wobei die zweite Nukleotidsequenz in Betriebsbeziehung mit einer zweiten Regulationsregion verknüpft ist, die in der Pflanze aktiv ist, und transient Coexprimieren der ersten und zweiten Nukleotidsequenz, um das Protein von Interesse zu synthetisieren, das weniger als 10 % Fucosylierung und Xylosylierung im Vergleich zu dem gleichen Protein von Interesse, das in einer Wildtyp-Pflanze produziert wird, umfasst.

2. Verfahren nach Anspruch 1, wobei die erste Regulationsregion ein Plastocyanin-Promotor oder ein 35S-Promotor ist, und die zweite Regulationsregion ein Plastocyanin-Promotor oder ein 35S-Promotor ist.

3. Verfahren nach Anspruch 1, wobei eine dritte Nukleotidsequenz in der Pflanze exprimiert wird, wobei die dritte Nukleotidsequenz für einen Silencing-Suppressor codiert, wobei die dritte Nukleotidsequenz in Betriebsbeziehung mit einer dritten Regulationsregion verknüpft ist, die in der Pflanze aktiv ist.

4. Verfahren nach Anspruch 3, wobei das Protein von Interesse in einer Menge von bis zu 1,5 g pro Kilogramm Pflanzenfrischgewicht synthetisiert wird.

5. Verfahren nach Anspruch 3, wobei die dritte Regulationsregion ein Plastocyanin-Promotor oder ein 35S-Promotor ist.

6. Verfahren nach Anspruch 2, wobei eine dritte Nukleotidsequenz in der Pflanze exprimiert wird, wobei die dritte Nukleotidsequenz für einen Silencing-Suppressor codiert, wobei die dritte Nukleotidsequenz in Betriebsbeziehung mit einer dritten Regulationsregion verknüpft ist, die in der Pflanze aktiv ist.

7. Verfahren nach Anspruch 6, wobei die dritte Regulationsregion ein Plastocyanin-Promotor oder ein 35S-Promotor ist.

8. Verfahren nach Anspruch 1, wobei das Protein von Interesse ein Antikörper, ein Antigen oder ein Vakzin ist.

9. Verfahren nach Anspruch 8, wobei die zweite Nukleotidsequenz, die für das Protein von Interesse codiert, eine Nukleotidsequenz 2A, die in Betriebsbeziehung mit einer Regulationsregion 2A verknüpft ist, die in der Pflanze aktiv ist, und eine Nukleotidsequenz 2B, die in Betriebsbeziehung mit einer Regulationsregion 2B verknüpft ist, die in der Pflanze aktiv ist, umfasst, und das Produkt, für das jeweils von 2A und 2B codiert wird, zusammen einen Antikörper produziert.

10. Verfahren nach Anspruch 9, wobei die Regulationsregion 2A ein Plastocyanin-Promotor ist und die Regulationsregion 2B ein Plastocyanin-Promotor ist.

11. Nukleinsäure, umfassend die Nukleotide 1 bis 1062 von SEQ: ID NO:17 (GNT1-GalT), wobei die Nukleotidsequenz für ein Protein codiert, welches die Glykosylierung eines Proteins von Interesse modifiziert.

12. Hybridprotein GNT1-GalT, umfassend eine CTS-Domäne von N-Acetylglucosaminyltransferase fusioniert an eine katalytische Domäne von beta-1,4-Galactosyltransferase, wobei das Hybridprotein Aminosäuren 1 bis 354 der Sequenz mit SEQ ID NO:18 umfasst.

13. Pflanze, Pflanzenzelle oder ein Samen, umfassend die Nukleotidsequenz nach Anspruch 11.

14. Pflanze, Pflanzenzelle oder ein Samen, umfassend das Hybridprotein nach Anspruch 12.

15. Verfahren nach Anspruch 3, wobei das Protein von Interesse ein Antikörper ist und in einer Menge von bis zu 1,5 g pro Kilogramm Blattfrischgewicht synthetisiert wird.

16. Verfahren nach Anspruch 1, wobei dem synthetisierten Protein von Interesse Fukosylierung, Xylosylierung oder beides fehlt.

## Revendications

1. Procédé de synthèse d'une protéine d'intérêt, avec une réduction de la fucosylation et de la xylosylation comprenant, l'introduction dans une plante, une partie d'une plante, ou une cellule végétale, d'une séquence nucléotidique comprenant de 80 % à 100 % d'identité avec une séquence nucléotidique définie par SEQ ID NO : 17 et codant pour une protéine hybride comprenant une queue cytoplasmique, un domaine transmembranaire, une région de tige (domaine CTS) de N-acétylglucosaminyltransférase (GNT1) fusionnée à un domaine catalytique de bêta-1,4-galactosyltransférase (GalT), la première séquence nucléotidique liée de façon fonctionnelle avec une première région régulatrice qui est active dans la plante, et d'une seconde séquence nucléotidique pour coder pour la protéine d'intérêt, la seconde séquence nucléotidique liée de façon fonctionnelle avec une seconde région régulatrice qui est active dans la plante, et la coexpression de façon transitoire des première et seconde séquences nucléotidiques pour synthétiser la protéine d'intérêt comprenant moins de 10 % de fucosylation et de xylosylation comparativement à la même protéine d'intérêt produite dans une plante de type sauvage.

2. Procédé selon la revendication 1, dans lequel la première région régulatrice est un promoteur de la plastocyanine, ou un promoteur 35S, et la seconde région régulatrice est un promoteur de la plastocyanine, ou un promoteur 35S.

3. Procédé selon la revendication 1, dans lequel une troisième séquence nucléotidique est exprimée au sein de la plante, la troisième séquence nucléotidique codant pour un suppresseur de silençage, la troisième séquence nucléotidique liée de façon fonctionnelle avec une troisième région régulatrice qui est active dans la plante.

4. Procédé selon la revendication 3, où la protéine d'intérêt est synthétisée dans une quantité de jusqu'à 1,5 g par kilogramme de poids frais de plante.

5. Procédé selon la revendication 3, dans lequel la troisième région régulatrice est un promoteur de la plastocyanine, ou un promoteur 35S.

6. Procédé selon la revendication 2, dans lequel une troisième séquence nucléotidique est exprimée au sein de la plante, la troisième séquence nucléotidique codant pour un suppresseur de silençage, la troisième séquence nucléotidique liée de façon fonctionnelle avec une troisième région régulatrice qui est active dans la plante.

7. Procédé selon la revendication 6, dans lequel la troisième région régulatrice est un promoteur de la plastocyanine, ou un promoteur 35S.

8. Procédé selon la revendication 1, dans lequel la protéine d'intérêt est un anticorps, un antigène ou un vaccin.

9. Procédé selon la revendication 8, dans lequel la seconde séquence nucléotidique codant pour la protéine d'intérêt comprend une séquence nucléotidique 2A, liée de façon fonctionnelle avec une région régulatrice 2A qui est active dans la plante, et une séquence nucléotidique 2B, liée de façon fonctionnelle avec une région régulatrice 2B qui est active dans la plante, et le produit codé par chacune de 2A et 2B se combine pour produire un anticorps.

10. Procédé selon la revendication 9, dans lequel la région régulatrice 2A est un promoteur de la plastocyanine, et la région régulatrice 2B est un promoteur de la plastocyanine.

11. Acide nucléique comprenant les nucléotides 1 à 1062 de SEQ ID NO : 17 (GNT1-GalT), dans lequel la séquence nucléotidique code pour une protéine qui modifie la glycosylation d'une protéine d'intérêt.

12. Protéine hybride GNT1-GalT, comprenant un domaine CTS de N-acétylglucosaminyltransférase fusionné à un domaine catalytique de bêta-1,4-galactosyltransférase, la protéine hybride comprenant les acides aminés 1 à 354 de SEQ ID NO : 18.

13. Plante, cellule végétale, ou graine comprenant la séquence nucléotidique selon la revendication 11.

14. Plante, cellule végétale, ou graine comprenant la protéine hybride selon la revendication 12.

15. Procédé selon la revendication 3, dans lequel la protéine d'intérêt est un anticorps et est synthétisée dans une quantité de jusqu'à 1,5 g par kilogramme de poids frais de feuilles.

16. Procédé selon la revendication 1, dans lequel la protéine d'intérêt synthétisée manque de fucosylation, de xylosylation ou des deux.
